(19) Europäisches Patentamt
European Patent Office
Office européen des brevets

(11) EP 1 789 170 B1

(12) **EUROPÄISCHE PATENTSCHRIFT**

(45) Veröffentlichungstag und Bekanntmachung des Hinweises auf die Patenterteilung:
**14.07.2010 Patentblatt 2010/28**

(21) Anmeldenummer: **05782622.4**

(22) Anmeldetag: **28.07.2005**

(51) Int Cl.:
***B01F 17/00*** *(2006.01)*

(86) Internationale Anmeldenummer:
**PCT/EP2005/008248**

(87) Internationale Veröffentlichungsnummer:
**WO 2006/018112 (23.02.2006 Gazette 2006/08)**

(54) **RHEOLOGIESTEUERUNG VON PICKERING-EMULSIONEN DURCH ELEKTROLYTE**

RHEOLOGY CONTROL OF PICKERING EMULSIONS BY ELECTROLYTES

CONTROLE DE LA RHEOLOGIE D'EMULSIONS DE PICKERING AU MOYEN D'ELECTROLYTES

(84) Benannte Vertragsstaaten:
**BE DE FR GB**

(30) Priorität: **12.08.2004 DE 102004039212**

(43) Veröffentlichungstag der Anmeldung:
**30.05.2007 Patentblatt 2007/22**

(73) Patentinhaber: **Wacker Chemie AG**
**81737 München (DE)**

(72) Erfinder: **GOTTSCHALK-GAUDIG, Torsten**
**84561 Mehring (DE)**

(74) Vertreter: **Gössmann, Christoph Tassilo et al**
**Wacker-Chemie GmbH**
**Zentralbereich PML,**
**Hanns-Seidel-Platz 4**
**81737 München (DE)**

(56) Entgegenhaltungen:
**WO-A-02/096378**
**WO-A-2005/092989**
**DE-A1- 10 238 649**
**US-A1- 2003 175 221**
**US-A1- 2003 175 317**
**US-B1- 6 585 983**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 789 170 B1

## Beschreibung

Die Erfindung betrifft den Einsatz von Elektrolyten zur Steuerung der Fließeigenschaften von Emulsionen vom Typ Öl-in-Wasser (O/W).

Emulsionen als Öl-in-Wasser- (O/W) Dispersion finden weite Verbreitung als Applikationsform für Beschichtungsstoffe wie z.B. wasserbasierende Farbe und Lacke, als Kleb- und Dichtstoffe wie z.B. wäßrige Epoxy- oder Polyurethansysteme, als kosmetische Formulierungen, als Reinigungs- und Desinfektionsmittel, in der Nahrungsmittelindustrie, zur Oberflächenmodifizierung fester Substrate oder als Reaktionsmedien bei der Emulsionspolymerisation.

Im allgemeinen erfolgt die Dispergierung und Stabilisierung der dispersen Phase mit Hilfe von Emulgatoren. Es werden kationische, anionische, ampholytische sowie nichtionische Emulgatoren verwendet. Den Emulgatoren ist gemeinsam, dass sie grenzflächenaktive Stoffe darstellen. D.h. sie lagern sich bevorzugt an Grenzflächen wie z.B. flüssig-flüssig, flüssigfest oder flüssig-gasförmig an und verringern dadurch die Grenzflächen- / Oberflächenenergie. Bei der Applikation der Emulsion können die Emulgatoren jedoch auch die Oberfläche des zu behandelnden Substrats bedecken und dabei die Benetzungseigenschaften der Oberfläche stark verändern. Dies kann z.B. die Adhäsionseigenschaften eines Beschichtungsstoffs oder einer Kleb- oder Dichtfuge negativ beeinflussen. Ferner kann die Wiederüberstreichbarkeit negativ beeinflusst werden. Zudem stellen Emulgatoren auf der Basis von organischen Molekülen potentielle Gefahrstoffe beim Einsatz in pharmazeutischen oder kosmetischen Formulierungen oder in Nahrungsmitteln dar.

Pickering beschrieb 1907 erstmalig die Herstellung von Emulsionen, die nur durch Zusatz verschiedener Feststoffe, wie basischer Kupfersulfate, basischer Eisensulfate oder anderer Metallsalze stabilisiert wurden. Diese Art von Emulsionen wird auch "Pickering-Emulsionen" genannt. Grundlegende Untersuchungen zeigten, dass ein Charakteristikum für Pickering-Emulsionen ist, daß Feststoffpartikel an der Grenzfläche zwischen den beiden flüssigen Phasen angeordnet sind und dort eine Barriere gegen die Koaleszenz der dispersen Phase bilden.

Dabei zeigte es sich, daß derartig Feststoff-stabilisierte Emulsionen, wie sie z.B. in DE 198 42 787 beschrieben wurden, zwar ausgesprochen stabil gegen Koaleszenz der dispersen (Öl-) Phase sind, jedoch insbesondere bei Phasenvolumina $\Phi$ der dispersen Phase größer oder gleich 0,5 hohe Viskositäten aufweisen, was negative Auswirkungen auf die Applikationseigenschaften der Emulsionen haben kann. Versuche, niedrige Viskositäten durch Verringerung des Phasenvolumens der dispersen Phase oder durch geringere Einsatzmengen an partikulärem Emulgator zu erreichen, führen häufig zu nicht ausreichend separationsstabilen Emulsionen.
Für den erfolgreichen Einsatz von Emulsionen beispielsweise als Beschichtungsstoff, ist die genaue Einstellung der Viskosität gemäß den Anforderungen der entsprechenden Applikation unerläßlich.

Aufgabe der Erfindung war es, die Nachteile des Stands der Technik zu überwinden.

Die Aufgabe wird durch die Erfindung gelöst.

Gegenstand der Erfindung sind Emulsionen nach Anspruch 1, die Emulsionen vom Typ Öl-in-Wasser (O/w) sind und enthaltend:

- eine Ölphase (Phase A), bestehend aus einer, gegebenenfalls mehreren, weitgehend wasserunlöslichen Komponente(n),
- eine Wasserphase (Phase B), die gegebenenfalls weitere wasserlösliche Komponenten wie organische Verbindungen wie Alkohole, Carbonsäuren oder andere Verbindungen enthalten kann
- an der Grenzfläche Öl-Wasser angeordnete pyrogene Kieselsäure, die in der Weise teilsilyliert ist, dass der Gehalt an nicht-silylierten Oberflächensilanolgruppen an der Siliciumdioxidoberfläche sich zwischen maximal 95% und minimal 50% des Ausgangs-Siliciumdioxids bewegt, gleichbedeutend mit 1,7 bis 0,9 SiOH-Gruppen pro $nm^2$ Kieselsäureoberfläche, der Dispersions-Anteil der Oberflächenenergie gamma-s-D 30 bis 80 $mJ/m^2$ beträgt, sowie die spezifische BET-Oberfläche einen Wert von 30 bis 500 $m^2/g$ aufweist,
- ein Elektrolytgehalt in der wäßrigen Phase, der so eingestellt ist , dass die Ionenstärke I der Lösung größer $10^{-6}$ mol/l ist, wobei die Ionenstärke definiert ist als $I = ½*\Sigma c_i*z_i^2$, wobei $c_i$ dabei die Konzentration des Ions i in der Lösung, $z_i$ die Ladung des Ions i ist,
- die mittlere Partikelgröße der dispersen Phase, d.h. der mittlere Tropfendurchmesser, gemessen mittels Laserbeugung, 0,5 $\mu$m bis 500 $\mu$m beträgt.
- und gegebenenfalls weitere Stoffe, wie Pigmente oder Konservierungsmittel.

Dabei war erstaunlich und für den Fachmann in keiner Weise vorherzusehen, dass durch die Verwendung von sinteraggregierter pyrogener Kieselsäure als partikulärer Emulgator und kleineren Mengen an Elektrolyt die rheologischen Eigenschaften der Emulsionen entsprechend den Bedürfnissen der Anwendung eingestellt werden können.

Sinteraggregate sind hier Sekundärstrukturen gemäß DIN 53206, die unter Scherbedingungen wie sie üblicherweise beim Dispergieren von Füllstoffen in flüssigen Medien wie z.B. lösungsmittelhaltigen oder lösungsmittelfreien Kleb- oder Beschichtungsstoffen auftreten, permanent sind, d.h. sich nicht in ihre Primärpartikel zerteilen lassen. Dies läßt sich beispielsweise an TEM-Aufnahmen gehärteter Kieselsäure-Bindemittel-Dispersionen nachweisen, die nur Aggregat-Strukturen aber keine isolierten Primärpartikel aufweisen.

Partikuläre Systeme bestehend aus Sinteraggregaten sind ferner dadurch charakterisiert, daß bei der Partikelgrößenbestimmung mittels quasielastischer Lichtstreuung erhaltene hydrodynamische Äquivalentdurchmesser mindestens um den Faktor 2 größer ist als der gemäß der Formel $a = 6/A_{BFT} * d$ rechnerisch erhältliche Durchmesser der Primärpartikel, wobei $A_{BET}$ die mittels Stickstoffadsorption gemäß DIN 66131 gemessene spezifische BET-Oberfläche und d die Dichte der Primärpartikel, ist.

Sinteraggregierte Systeme sind ferner dadurch charakterisiert, daß die fraktale Dimension df der Masse vorzugsweise kleiner 2,7 ist, wobei die fraktale Dimension df definiert ist als Masse proportional zum Radius R hoch df. Die fraktale Dimension der Masse läßt sich beispielsweise mittels Röntgen- oder Neutronen-Kleinwinkel-Streuung bestimmen.

Bevorzugt sind die erfindungsgemäßen Emulsionen im wesentlichen frei von herkömmlichen, bei Raumtemperatur und dem Druck der umgebenden Atmosphäre nicht partikulären flüssigen und festen, rein organischen oberflächenaktiven Substanzen, wie nichtionischen, kationischen und anionischen Emulgatoren. Nicht-partikuläre Emulgatoren heißt hier keine Partikel und Kolloide, sondern Moleküle und Polymere, folgend der Definition von Molekülen, Polymeren, Kolloide und Partikel wie gegeben in 'Dispersionen und Emulsionen', G. Lagaly, O. Schulz, R. Zindel, Steinkopff, Darmstadt 1997, ISBN 3-7985-1087-3, S. 14.

Im allgemeinen weisen diese organischen Emulgatoren eine Größe kleiner 1 nm, eine Molmasse < 10000 g/mol, einen Kohlenstoffgehalt > 50 Gew.-%, bestimmbar durch Elementaranalyse, sowie eine Mohs'sche Härte kleiner als 1 auf.

Zugleich weisen die Emulgatoren, von denen die erfindungsgemäßen Emulsionen vorzugsweise im wesentlichen frei sind, zumeist eine Löslichkeit in Wasser bei 20 °C und dem Druck der umgebenden Atmosphäre, also 900 bis 1100 hPa, homogen oder in Micellen-Form von größer 1 Gew.-% auf. Die erfindungsgemäßen Emulsionen können solche oberflächenaktive Substanzen bis zu einer maximalen Konzentration von kleiner als 0,1 mal, bevorzugt kleiner als 0,01 mal, besonders bevorzugt kleiner als 0,001 mal, insbesondere kleiner als 0,0001 mal der kritischen Micellkonzentration dieser oberflächenaktiven Stoffe in der Wasserphase enthalten; dies entspricht einer Konzentration dieser oberflächenaktiven Substanzen, bezogen auf das Gesamtgewicht der erfindungsgemäßen Emulsion von kleiner 10 Gew.-%, bevorzugt kleiner 2 Gew.-%, besonders bevorzugt kleiner 1 Gew.-%, insbesondere 0 Gew.-%.

Die erfindungsgemäße Emulsion enthält eine Ölphase (Phase A). Phase A besteht aus einer, gegebenenfalls mehreren, weitgehend wasserunlöslichen Komponente(n). Weitgehend wasserunlöslich bedeutet hier, dass die Löslichkeit der Komponenten in Wasser alleine oder als Mischung kleiner 10 g/100 g Wasser, bevorzugt kleiner 1 g/100 g Wasser, besonders bevorzugt kleiner 0,1 g/100 g Wasser ist, gemessen bei 20 °C und dem Druck der umgebenden Atmosphäre, also 900 bis 1100 hPa ist.

In der erfindungsgemäßen Emulsion beträgt die Viskosität der Phase A, gemessen bei 20 °C und einem Schergefälle von 10 $s^{-1}$, 0,1 bis 1000000 mPa·s, bevorzugt 0,1 bis 500000 mPa·s, besonders bevorzugt 0,2 bis 100000 mPa·s.

In der erfindungsgemäßen Emulsion kann die Phase A vorzugsweise mehrere Komponenten enthalten. Bei den Einzelkomponenten kann es sich dabei sowohl um bei 20 °C flüssige Stoffe als auch um Feststoffe handeln, wobei die Gesamtmischung der Einzelkomponenten die oben genannt Viskosität aufweist. Bevorzugt, jedoch nicht notwendigerweise, handelt es sich bei einer mehrkomponentigen Phase A um eine echte Lösung, d.h. um eine homogene Phase bei der keine weiteren Phasengrenzflächen auftreten.

Beispiele für weitgehend wasserunlösliche Komponenten wie sie die Phase A einer erfindungsgemäßen Emulsion bilden oder in ihr enthalten sein können sind aliphatische und aromatische Kohlenwasserstoffe, Alkohole, Aldehyde, Ketone, Ether, Ester, Amine, Carbonsäuren und deren Derivate, Mercaptane, Thioether, oligomere bzw. polymere Verbindungen wie Polyolefine, wie Polystyrole, Polypropylene oder Polyethylene, gesättigte oder ungesättigte Polyester wie z.B. Polykondensate aus Phthalsäuren und 1,2-Propandiolen bzw. Poly-co-kondensate aus Phthalsäuren, 1,2-Propandiolen und Maleinsäuren gegebenenfalls gelöst in Reaktivverdünnern wie Styrolen, Polyether, Polyepoxide, bzw. deren monomeren oder oligomeren Vorstufen, wie Alkylenbisglycidylether, wie

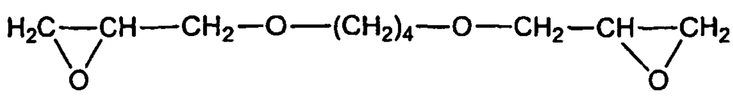

Bisphenol A basierende Diglycidylether, wie

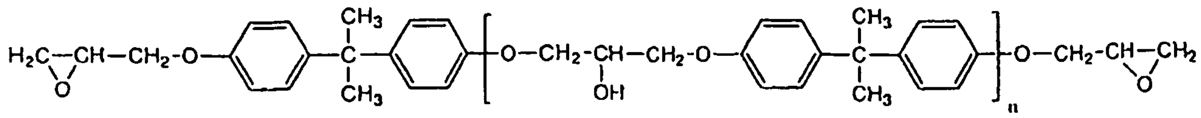

mit n bevorzugt von 0 bis 10, besonders bevorzugt 0 bis 5

Beispiele für Epoxy-Novolac-Harze sind solche der Formel

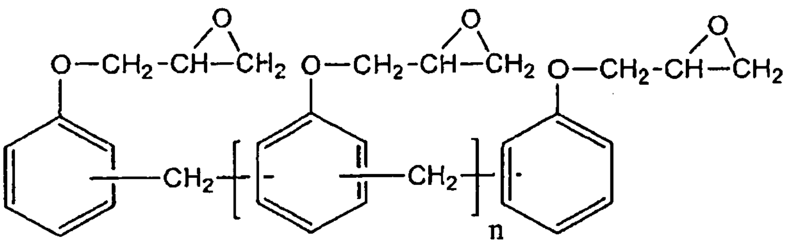

bifunktionelle Epoxyverbindungen, wie

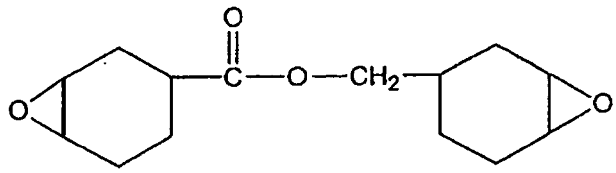

trifunktionelle Epoxyverbindungen, wie

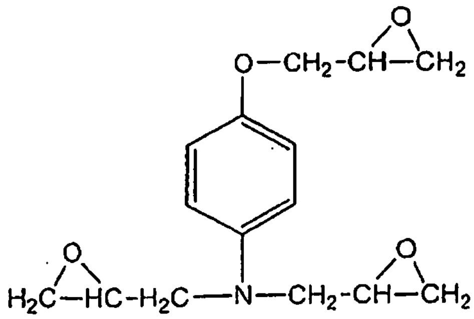

tetrafunktionelle Epoxyverbindungen, wie

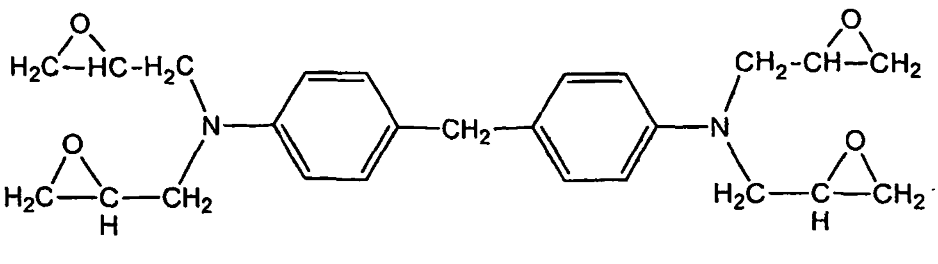

Polyurethane bzw. deren monomeren oder oligomeren Vorstufen wie z.B. Polyetherpolyole, Polyacrylatpolyole, Polyesterpolyole, multifunktionelle Isocyanate wie Hexandiisocyanat, Toluendiisocyanat, Diphenylmethandiisocyanat bzw. mit blockierenden Schutzgruppenversehene Isocyanate wie Hydroxylamine oder Malonesterderivate, komplexe organische Verbindungen wie künstliche bzw. natürliche pharmazeutische oder kosmetische Wirkstoffe, Farbstoffe, elementorganische Verbindungen wie Organosiliziumverbindungen, wie Organo (poly) silane, Organo (poly) siloxane, Organo (poly) silazane und Organo(poly)silcarbane, oder Übergangsmetallverbindungen. Gegebenenfalls kann Phase A ölbenetzbare Partikel wie Pigmente, Füllstoffe oder rheologische Additive enthalten.

Die erfindungsgemäße Emulsion enthält ferner eine wässrige Phase (Phase B). Phase B kann neben Wasser weitere Komponenten enthalten, wie vorzugsweise Säuren, Basen, wasserlösliche organische Verbindungen, wie Alkohole, Carbonsäuren und deren Derivate, Amine oder andere organische Verbindungen, polymere oder oligomere Verbindungen wie Polyole oder Polyamine bzw. Polyamidoamine, komplexe wasserlösliche organische Verbindungen, wie künstliche bzw. natürliche pharmazeutische oder kosmetische Wirkstoffe, Farbstoffe, elementorganische Verbindungen wie wasserlösliche Organosiliziumverbindungen oder wasserlösliche Übergangsmetallverbindungen. Gegebenenfalls kann Phase B wasserbenetzbare Partikel wie Pigmente, Füllstoffe oder rheologische Additive enthalten.

Zur Einstellung der benötigten Viskosität der Emulsion können der Wasserphase B ferner organische oder anorganische (Poly)-Elektrolyte in der Weise zugesetzt werden, daß die Ionenstärke in der wäßrigen Phase vorzugsweise kleiner 160 mol/l, bevorzugt $10^{-6}$ mol/l bis 16 mol/l, besonders bevorzugt $10^{-5}$ mol/l bis 1,6 mol/l und ganz besonders bevorzugt $10^{-4}$ mol/l bis 0,8 mol/l beträgt, wobei die Ionenstärke definiert ist als $I = ½*\Sigma c_i*z_i^2$, $c_i$ ist dabei die Konzentration des Ions i in der Lösung, $z_i$ die Ladung des Ions i.

Die erfindungsgemäßen Emulsionen enthalten an der Grenzfläche Öl-Wasser angeordnete Sinteraggregate geeigneter pyrogener Kieselsäuren. Bei den erfindungsgemäß eingesetzten Sinteraggregaten handelt es sich um zum Teil mit Wasser benetzbare Sinteraggregate, d.h. die nicht vollständig mit Wasser benetzbar sind und nicht vollständig Wasser-unbenetzbar sind.

Bei den erfindungsgemäß eingesetzten Sinteraggregaten handelt es sich um bei Raumtemperatur und dem Druck der umgebenden Atmosphäre, also zwischen 900 und 1100 hPa, feste Sinteraggregate.

Die erfindungsgemäß eingesetzten Sinteraggregate weisen bevorzugt eine Löslichkeit in Wasser bei pH 7,33 und einem Elektrolythintergrund von 0,11 Mol und einer Temperatur von 37°C von kleiner 0,1 g/l, besonders bevorzugt von kleiner als 0,05 g/l, auf, bei dem Druck der umgebenden Atmosphäre, also zwischen 900 und 1100 hPa.

Bevorzugt weisen die erfindungsgemäß eingesetzten Sinteraggregate einen mittleren hydrodynamischen Äquivalenzdurchmesser $D_h$ größer 1 nm, vorzugsweise von 1 bis 5000 nm, bevorzugt von 10 bis 1000 nm, besonders bevorzugt von 100 bis 600 nm, ganz besonders bevorzugt 200 nm bis 500 nm, speziell ausgewählt von 210 nm bis 450 nm auf, jeweils gemessen bevorzugt mittels dynamischer Lichtstreuung.

Dies bedeutet, dass der für die Ausbildung einer Partikelschicht in der Öl-Wasser-Grenzfläche relevante Kollisionsradius $R_c$ der Sinteraggregate größer 0,8 nm, vorzugsweise 0,8 bis 4000 nm, bevorzugt 8 bis 850 nm, besonders bevorzugt 80 bis 500 nm, ganz besonders bevorzugt 170 nm bis 375 nm ist. Der Kollisionsradius ist dabei der Radius der kleinsten Kugel, die gerade noch alle Bestandteile eines Aggregates einschließt, wobei sich der Kollisionsradius $R_c$ ergibt aus der Gleichung $R_c = [R_h^2/0,76 + 2,63 * R_h^2/d_f]^{0,5}$ wie gegeben in R. de Rooij, A. A. Potanin, D. van den Ende, J. Mellema, J. Chem. Phys. 1993, 99, 9213, wobei der hydrodynamische Äquivalenzradius $R_h$ erhalten wird aus hydrodynamische Äquivalenzdurchmesser dividiert durch 2 und die fraktale Dimension der Masse $d_f$ einen Wert von 1,8 aufweist.

Bevorzugt sind die erfindungsgemäß eingesetzten Sinteraggregate ferner dadurch charakterisiert, daß bei der Partikelgrößenbestimmung mittels quasielastischer Lichtstreuung der hydrodynamische Äquivalentdurchmesser mindestens um dem Faktor 2, bevorzugt um den Faktor 5 bis 50, besonders bevorzugt um den Faktor 7 bis 25, ganz besonders bevorzugt um den Faktor 7,5 bis 16,5, jeweils bezogen auf eine spezifische Oberfläche von 100 $m^2$/g, bei kleinerer bzw. größerer Oberfläche verkleinert bzw. vergrößert sich der Faktor entsprechend linear, größer ist als der gemäß der Formel $a = 6/A_{BET} * d$ rechnerisch erhältliche Durchmesser der Primärpartikel, wobei $A_{BET}$ die mittels Stickstoffadsorption gemäß DIN 66131 gemessene spezifische BET-Oberfläche und d die Dichte der Primärpartikel ist.

Bevorzugt weisen die erfindungsgemäß eingesetzten Sinteraggregate eine Molmasse größer 10 000 g/Mol, besonders bevorzugt eine Molmasse von 50 000 bis 50 000 000 g/Mol, insbesondere von 100 000 bis 10 000 000 g/Mol, auf, jeweils gemessen bevorzugt mittels statischer Lichtstreuung.

Bevorzugt weisen die erfindungsgemäß eingesetzten Sinteraggregate eine spezifische BET-Oberfläche von 30 bis 500 $m^2$/g, besonders bevorzugt 80 bis 300 $m^2$/g, auf. Die BET-Oberfläche wird nach bekannten Verfahren gemessen, bevorzugt gemäß Deutscher Industrie Norm DIN 66131 und DIN 66132.

Bevorzugt weisen die erfindungsgemäß eingesetzten Sinteraggregate einen Kohlenstoffgehalt von kleiner 50 Gewichtsprozent auf.

Bevorzugt weisen die erfindungsgemäß eingesetzten Sinteraggregate eine Mohs'sche Härte größer als 1, besonders bevorzugt größer als 4, auf.

Vorzugsweise weisen die Sinteraggregate eine Oberflächenenergie gamma von 30 bis 72,5 $mJ/m^2$, bei einer

Temperatur von 25°C und dem Druck der umgebenden Atmosphäre, also zwischen 900 und 1100 hPa, auf.

Vorzugsweise weisen die erfindungsgemäß eingesetzten Kieselsäure-Sinteraggregate einen Dispersions-Anteil der Oberflächenenergie gamma-s-D von 30 bis 80 $mJ/m^2$, bevorzugt 35 bis 70 $mJ/m^2$, besonders bevorzugt 40 bis 70 $mJ/m^2$, bei einer Temperatur von 25°C und dem Druck der umgebenden Atmosphäre, also zwischen 900 und 1100 hPa, auf. Gemessen wird der Dispersions-Anteil der Oberflächenenergie gamma-s-D zum Beispiel gemäß "Inverse Gaschromatography"-"Characterisation of Polymers and other Materials", 391 ACS Symposium Series, D R Lloyd, Th C Ward, H P Schreiber, Chapter 18, pp 248-261, ACS, Washington DC 1989, ISBN 0-8412-1610-X.

Die bevorzugte Ausgangs-Kieselsäure, aus der die in den erfindungsgemäßen Emulsionen eingesetzte, zum Teil mit Wasser benetzbare Kieselsäure hergestellt wird, kann auf beliebige an sich bekannte Weise, wie beispielsweise in einer Flammenreaktion aus Halogensiliciumverbindungen hergestellt werden, z.B. aus Siliciumtetrachlorid oder Halogen-Organosiliciumverbindungen, wie Methylchlorsilanen, wie Methyltrichlorsilan, oder Hydrogenchlorsilanen, wie Hydrogentrichlorsilan oder anderen Hydrogenmethylchlorsilanen, wie Hydrogenmethyldichlorsilan oder Alkylchlorsilanen, auch im Gemisch mit Kohlenwasserstoffen, oder beliebigen versprühbaren und, bevorzugt, verflüchtigbaren Gemischen aus Organosiliciumverbindungen, wie genannt, und Kohlenwasserstoffen, wobei es sich bei der Flamme um eine Wasserstoff-Sauerstoff-Flamme oder auch eine Kohlenmonoxid-Sauerstoff-Flamme handeln kann. Die Herstellung der Kieselsäure kann dabei wahlweise mit und ohne zusätzlichem Zusatz von Wasser erfolgen, zum Beispiel im Schritt der Reinigung; bevorzugt ist kein Zusatz von Wasser.

Bevorzugt werden zur Herstellung der erfindungsgemäßen Emulsionen als Kieselsäure-Sinteraggregate teilhydrophobierte, besonders bevorzugt teilsilylierte, Kieselsäure-Sinteraggregate eingesetzt.

Teilweise silyliert bedeutet hier, dass weder die gesamte Kieselsäure-Oberfläche unsilyliert ist, noch dass die gesamte Kieselsäure-Oberfläche silyliert ist.

Der Bedeckungsgrad $\tau$ der Oberfläche der Kieselsäure-Sinteraggregate mit Silyliermittelresten ist dabei, bezogen auf die Gesamt-Partikel-Oberfläche, bevorzugt 5 bis 95 %, besonders bevorzugt 10 bis 90 %, insbesondere 15 bis 75 %.

Die Bedeckung mit Silyliermittel kann dabei beispielsweise mittels Elementaranalyse, wie z.B. über den Kohlenstoffgehalt, ermittelt werden oder durch Bestimmung des Rest-Gehaltes an reaktiven Oberflächen-Silanol-Gruppen der Kieselsäure-Sinteraggregate.

Teilsilylierung bedeutet dabei, daß der Kohlenstoffgehalt der Kieselsäure 0,1 bis 10 Gew-%, bevorzugt 0,1 bis 5 Gew-%, besonders bevorzugt 0,1 bis 3 Gew-%, insbesondere 0,1 bis 1,5 Gew-% beträgt.

Teilsilylierung bedeutet ferner, dass der Gehalt an nicht-silylierten Oberflächensilanolgruppen an der

Siliciumdioxidoberfläche sich zwischen bevorzugt maximal 95 % und minimal 50 %, besonders bevorzugt von 90 bis 60 %, insbesondere 85 bis 65 %, des Ausgangs-Siliciumdioxids bewegt.

Dies bedeutet, dass die Dichte der Oberflächensilanolgruppen SiOH sich vorzugsweise zwischen minimal 0,9 und maximal 1,7, bevorzugt von 1,1 bis 1,6, besonders bevorzugt von 1,2 bis 1,55, SiOH pro $nm^2$ Partikeloberfläche bewegt.

Für ein Ausgangs-Siliciumdioxid von 200 $m^2/g$ spezifischer Oberfläche, das zur Silylierung herangezogen werden kann, bedeutet dies vorzugsweise zwischen minimal 0,3 mMol/g SiOH und maximal 0,57 mMol/g SiOH, bevorzugt 0,36 bis 0,54 mMol/g SiOH, besonders bevorzugt 0,39 bis 0,51 mMol/g SiOH; für ein Siliciumdioxid mit geringerer bzw. größerer Oberfläche bedeutet dies linear proportional mehr oder weniger Oberflächensilanolgruppen SiOH.

Verfahren zur Teilhydrophobierung bzw. Teilsilylierung von Feststoffpartikel sind bereits bekannt.

Vorzugsweise weist die Ausgangs-Kieselsäure eine spezifische BET-Oberfläche von 25 bis 500 $m^2/g$ auf. Die Ausgangs-Kieselsäure weist vorzugsweise Sinteraggregate (Definition nach DIN 53206) im Bereich von Durchmessern 200 bis 1000 nm auf, wobei die Kieselsäure aus Sinteraggregaten aufgebaute Agglomerate (Definition nach DIN 53206) aufweist, die in Abhängigkeit von der äußeren Scherbelastung (z.B. Messbedingungen) Größen von 1 bis 500 $\mu m$ aufweisen.

Vorzugsweise weist die Ausgangs-Kieselsäure eine fraktale Dimension der Oberfläche von vorzugsweise kleiner oder gleich 2,3 auf, wobei die fraktale Dimension der Oberfläche $D_s$ hierbei definiert ist als: Partikel-Oberfläche A ist proportional zum Partikel-Radius R hoch $D_s$. Vorzugsweise weist die Ausgangs-Kieselsäure eine Dichte an zugänglichen, d.h. einer chemischen Reaktion zugänglichen, Oberflächen-Silanolgruppen SiOH von bevorzugt 1,5 bis 2,5 SiOH pro $nm^2$ spezifischer Oberfläche, besonders bevorzugt 1,6 bis 2,0 SiOH pro $nm^2$, auf.

Zur Herstellung der erfindungsgemäß eingesetzten Kieselsäure-Sinteraggregate können als Ausgangs-Kieselsäuren bei hoher Temperatur (größer 1000°C) hergestellte Kieselsäuren eingesetzt werden, wobei pyrogen hergestellte Kieselsäuren besonders bevorzugt sind. Es können hydrophile Kieselsäuren eingesetzt werden, die frisch hergestellt direkt aus dem Brenner kommen, zwischengelagert oder bereits handelsüblich verpackt sind.

Als Ausgangs-Kieselsäuren können unverdichtete, mit Stampf- oder Klopfdichten kleiner 60 g/l, aber auch verdichtete, mit Stampf- oder Klopfdichten größer 60 g/l, Kieselsäuren eingesetzt werden.

Als Ausgangs-Kieselsäuren können Gemische aus verschiedenen Kieselsäuren eingesetzt werden, so z.B. Mischungen aus Kieselsäuren unterschiedlicher BET-Oberfläche.

Bevorzugt können zur Silylierung von Kieselsäuren Organosiliciumverbindungen, wie z.B.

(i) Organosilane bzw. Organosilazane der Formel

$$R^1_dSiY_{4-d} \qquad (I)$$

und/oder deren Teilhydrolysate,
wobei
$R^1$ gleich oder verschieden sein kann und einen einwertigen, gegebenenfalls substituierten, gegebenenfalls einfach oder mehrfach ungesättigten, gegebenenfalls aromatischen Kohlenwasserstoffrest mit 1 bis 24 Kohlenstoff-Atomen, der durch Sauerstoffatome unterbrochen sein kann, bedeutet,
d gleich 1, 2 oder 3 bedeutet und
Y gleich oder verschieden sein kann und Halogenatom, einwertige Si-N-gebundene Stickstoffreste, an den ein weiterer Silylrest gebunden sein kann, $-OR^2$ oder $-OC(O)OR^2$ bedeutet, wobei $R^2$ gleich Wasserstoffatom oder ein einwertiger, gegebenenfalls substituierter, gegebenenfalls einfach oder mehrfach ungesättigten Kohlenwasserstoffrest, der durch Sauerstoffatome unterbrochen sein kann, bedeutet,
oder
(ii) lineare, verzweigte oder cyclische Organosiloxane aus Einheiten der Formel

$$R^3_e(OR^4)_fSiO_{(4-e-f)/2} \qquad (II),$$

wobei
$R^3$ gleich oder verschieden sein kann und eine der oben für $R^1$ angegebenen Bedeutungen hat,
$R^4$ gleich oder verschieden sein kann und eine für $R^3$ angegebene Bedeutung hat,
e 0, 1, 2 oder 3 ist und
f 0, 1, 2, 3 ist, mit der Maßgabe dass die Summe $e+f \leq 3$ ist, oder
Gemische aus (i) und (ii)
eingesetzt werden.

Bei den Organosiliciumverbindungen, die zur Silylierung der Kieselsäuren eingesetzt werden können, kann es sich beispielsweise um Gemische aus Silanen oder Silazanen der Formel (I) handeln, wobei solche aus Methyl-Chlorsilanen einerseits oder Alkoxysilanen und gegebenenfalls Disilazanen andererseits bevorzugt sind.

Beispiele für $R^1$ sind die oben angegebenen Reste, wobei es sich bevorzugt um den Methyl-, Octyl-, Phenyl- und Vinylrest handelt, besonders bevorzugt um den Methylrest.

Beispiele für $R^2$ sind vorzugsweise der Methyl-, der Ethyl-, der Propyl und der Octylrest, wobei der Methyl- und der Ethylrest bevorzugt sind.

Beispiele für Organosilane der Formel (I) sind Alkylchlorsilane, wie Methyltrichlorsilan, Dimethyldichlorsilan, Trimethylchlorsilan, Octylmethyldichlorsilan, Octyltrichlorsilan, Octadecylmethyldichlorsilan und Octadecyltrichlorsilan, Methylmethoxysilane, wie Methyltrimethoxysilan, Dimethyldimethoxysilan und Trimethylmethoxysilan, Methylethoxysilane, wie Methyltriethoxysilan, Dimethyldiethoxysilan und Trimethylethoxysilan, Methylacetoxysilane, wie Methyltriacetoxysilan, Dimethyldiacetoxysilan und Trimethylacetoxysilan, Phenylsilane, wie Phenyltrichlorsilan, Phenylmethyldichlorsilan, Phenyldimethylchlorsilan, Phenyltrimethoxysilan, Phenylmethyldimethoxysilan, Phenyldimethylmethoxysilan, Phenyltriethoxysilan, Phenylmethyldiethoxysilan und Phenyldimethylethoxysilan Vinylsilane, wie Vinyltrichlorsilan, Vinylmethyldichlorsilan, Vinyldimethylchlorsilan, Vinyltrimethoxysilan, Vinylmethyldimethoxysilan, Vinyldimethylmethoxysilan, Vinyltriethoxysilan, Vinylmethyldiethoxysilan und Vinyldimethylethoxysilan, Disilazane wie Hexamethyldisilazan, Divinyltetramethyldisilazan und Bis(3,3-trifluorpropyl)tetramethyldisilazan, Cyclosilazane wie Octamethylcyclotetrasilazan, und Silanole wie Trimethylsilanol.

Bevorzugt ist Methyltrichlorsilan, Dimethyldichlorsilan und Trimethylchlorsilan oder Hexamethyldisilazan.

Beispiele für Organosiloxane der Formel (II) sind lineare oder cyclische Dialkylsiloxane mit einer mittleren Anzahl an Dialkylsiloxyeinheiten von größer als 3. Die Dialkylsiloxane sind bevorzugt Dimethylsiloxane. Besonders bevorzugt sind lineare Polydimethylsiloxane mit folgenden Endgruppen: Trimethylsiloxy-, Dimethylhydroxysiloxy-, Dimethylchlorsiloxy-, Methyldichlorsiloxy-, Dimethylmethoxysiloxy-, Methyldimethoxysiloxy-, Dimethylethoxysiloxy-, Methyldiethoxysiloxy-, Dimethylacetoxysiloxy-, Methyldiacetoxysiloxy- und Dimethylhydroxysiloxygruppen, insbesondere mit Trimethylsiloxy- oder Dimethylhydroxysiloxyendgruppen.

Bevorzugt haben die genannten Polydimethylsiloxane eine Viskosität bei 25°C von 2 bis 100 mPa·s.

Weitere Beispiele für Organosiloxane sind Siliconharze, im besonderen solche, die als Alkylgruppen Methylgruppen enthalten, wobei es sich besonders bevorzugt um solche handelt, die $R^3_3SiO_{1/2}$ und $SiO_{4/2}$-Einheiten enthalten oder solche, die $R^3SiO_{3/2}$ und gegebenenfalls $R^3_2SiO_{2/2}$-Einheiten enthalten, wobei $R^3$ eine der oben genannten Bedeutungen hat.

Bevorzugt haben die genannten Siliconharze aus Einheiten der Formel (II) eine Viskosität bei 25°C von 500 bis 5000 $mm^2/s$.

Bei Siliconharzen mit einer Viskosität von größer als 1000 $mm^2/s$ bei 25°C sind solche bevorzugt, die sich in einem technisch gut handhabbaren Lösungsmittel, wie vorzugsweise Alkohole wie Methanol, Ethanol, iso-Propanol, Ether wie Diethylether, Tetrahydrofuran, Siloxane wie Hexamethyldisiloxan, Alkane wie Cyclohexan oder n-Octan, Aromaten wie Toluol oder Xylol, mit einer Konzentration über 10 Gew.-% und einer Mischungsviskosität kleiner als 1000 $mm^2/s$ bei einer Temperatur von 25°C und dem Druck der umgebenden Atmosphäre lösen lassen.

Unter den festen Organosiloxanen sind solche bevorzugt, die sich in einem technisch handhabbaren Lösungsmittel (wie oben definiert) mit einer Konzentration größer als 10 Gew.% und einer Mischungsviskosität kleiner als 1000 $mm^2/s$ bei einer Temperatur von 25°C lösen.

Die Hydrophobierung und Silylierung, die zur Herstellung der erfindungsgemäß eingesetzten Kieselsäure-Sinteraggregate bevorzugt durchgeführt wird, kann als diskontinuierliche Reaktion, d.h. im Batch-Verfahen, oder als kontinuierliche Reaktion durchgeführt werden, wobei die kontinuierliche Reaktion bevorzugt ist.

Die Hydrophobierung und Silylierung kann in einem Schritt realisiert werden oder in 2 oder 3 aufeinander folgenden Schritten. Das heißt, der Reaktion kann eine Beladung (Physisorption des Silyliermittels) vorgeschaltet sowie der Reaktion vorzugsweise ein Reinigungsschritt nachgeschaltet sein. Bevorzugt sind 3 suksessive Schritte: (1) Beladung - (2) Reaktion - (3) Reinigung.

Die Beladungstemperatur liegt bei vorzugsweise -30 bis 350°C, bevorzugt 20 bis 120°C.

Die Reaktionstemperaturen reichen vorzugsweise von 0 bis 400°C, besonders bevorzugt von 20 bis 330°C.

Die Reaktionszeiten dauern vorzugsweise von 1 Minute bis 24 Stunden, bevorzugt 30 Minuten bis 4 Stunden.

Der Reaktionsdruck liegt vorzugsweise im Bereich Normaldruck, d.h. zwischen 900 und 1100 hPa.

Die Reinigungstemperatur reicht vorzugsweise von 80 bis 400°C.

Eine effektive Bewegung und Durchmischung von Kieselsäure und Silyliermittel während Schritte (1) Beladung, (2) Reaktion und Reinigung (3) ist notwendig. Dies erfolgt bevorzugt durch mechanische oder gasgetragene Fluidisierung. Eine gasgetragene Fluidisierung kann durch alle inerten Gase erfolgen, die nicht zu Nebenreaktionen, Abbaureaktionen, Oxidationsvorgängen und Flammen- und Explosionserscheinungen führen. Die Leerrohrgasgeschwindigkeit ist hierbei von 0,05 bis 5 cm/s, besonders bevorzugt von 0,05 bis 1 cm/s. Mechanische Fluidisierung kann durch Flügelrührer, Ankerrührer und sonstige geeignete Rührorgane erfolgen.

In einer besonders bevorzugten Ausführung wird nur die Gasmenge zugeführt, die zur Aufrechterhaltung einer sauerstoffarmen Atmosphäre ausreicht, bevorzugt weniger als 5 Vol.-%, die Fluidisierung erfolgt dann rein mechanisch.

Die Reaktion wird bevorzugt in einer Atmosphäre durchgeführt, die nicht zur Oxidation der silylierten Kieselsäure führt, d.h. bevorzugt weniger als 10 Vol.-% Sauerstoff, besonders bevorzugt sind weniger als 2,5 Vol.-%, wobei beste Ergebnisse bei weniger als 1 Vol.-% Sauerstoff erzielt werden.

Es erfolgt ein effektives Einbringen der Silyliermittel in die Kieselsäure. Handelt es sich bei den Silyliermitteln bei Applikationstemperatur um flüssige Verbindungen, werden bevorzugt effektive Verdüsungstechniken eingesetzt. Verdüsen in 1-Stoffdüsen unter Druck (5 bis 20 bar), Versprühen in 2-Stoffdüsen unter Druck (Gas und Flüssigkeit 2 bis 20 bar), Feinstverteilen mit Atomizern, etc.

Bevorzugt wird das Silyliermittel als feinstverteiltes Aerosol zugefügt, wobei das Aerosol eine Sinkgeschwindigkeit von vorzugsweise 0,1 bis 20 cm/s und eine Tropfengröße mit einem aerodynamischen Äquivalentdurchmesser von 5 bis 25 $\mu m$ aufweist.

Wahlweise können vorzugsweise protische Lösemittel hinzugefügt werden, wie flüssige oder verdampfbare Alkohole oder Wasser; typische Alkohole sind iso-Propanol, Ethanol und Methanol. Es können auch Gemische der oben genannten protischen Lösemittel zugefügt werden. Bevorzugt werden keine protischen Lösemittel zugesetzt.

Wahlweise können vorzugsweise saure oder basische Katalysatoren zugesetzt werden. Diese Katalysatoren können basischen Charakters sein, im Sinne einer Lewis Base oder einer Brönsted Base, wie Ammoniak, oder sauren Charakters sein, im Sinne einer Lewis Säure oder einer Brönsted Säure, wie Chlorwasserstoff. Falls Katalysatoren eingesetzt werden, handelt es sich bevorzugt um Spuren, d.h weniger als 1000 ppm. Besonders bevorzugt werden keine Katalysatoren zugesetzt.

Der Reinigungsschritt ist durch Bewegung gekennzeichnet, wobei langsame Bewegung und geringes Durchmischen bevorzugt ist.

Der Reinigungsschritt ist weiterhin durch erhöhten Gaseintrag gekennzeichnet, entsprechend einer Leerrohrgasgeschwindigkeit von 0,001 bis 10 cm/s.

Zusätzlich kann der Reinigungsschritt ein Mischen mit mechanischen Rührorganen beinhalten. Die Rührorgane werden dabei so eingestellt und bewegt, dass bevorzugt Mischen und Fluidisieren, jedoch nicht völlige Verwirbelung eintritt.

Zusätzlich können während des Silylierschrittes Verfahren zur mechanischen Verdichtung eingesetzt werden, wie zum Beispiel Presswalzen, Kugelmühlen, Kollergänge, Schraubenverdichter und Brikettierer.

Zusätzlich können vor, während oder nach des

Silylierungsschrittes Verfahren zur Desagglomerierung der Kieselsäure eingesetzt werden, wie Stiftmühlen oder Vorrichtungen zur Mahlsichtung und/oder Verfahren zur mechanischen Verdichtung der Kieselsäure, wie zum Beispiel Presswalzen, oder Verdichten durch Absaugen des Luft- oder Gasinhaltes durch geeignete Vakuummethoden oder andere Verfahren zur mechanischen Verdichtung wie zum Beispiel Presswalzen, Kugelmühlen, Kollergänge, Schraubenverdichter und Brikettierer.

Die Herstellung der Kieselsäure-Sinteraggregate kann auch insitu bei der Herstellung der erfindungsgemäßen Emulsionen erfolgen.

Die erfindungsgemäßen Emulsionen enthalten Kieselsäure-Sinteraggregate in Mengen von bevorzugt 0,1 bis 50 Gewichtsteilen, besonders bevorzugt 1 bis 15 Gewichtsteilen, insbesondere 2 bis 10 Gewichtsteilen, bezogen auf 100 Gewichtsteile Gesamtemulsion.

Bei den erfindungsgemäßen Emulsionen kann der Volumenbruch $\Phi_o$ der Ölphase definiert als $\Phi_o$ = Volumen Ölphase / (Volumen Ölphase + Volumen Wasserphase) vorzugsweise 0,1 bis 0,9, bevorzugt 0,2 bis 0,8, besonders bevorzugt 0,3 bis 0,75, insbesondere 0,4 bis 0,7 betragen.

Bei den erfindungsgemäßen Emulsionen kann der Volumenbruch $\Phi_w$ der Wasserphase definiert als $\Phi_w$ = Volumen Wasserphase / (Volumen Ölphase + Volumen Wasserphase) vorzugsweise 0,1 bis 0,9, bevorzugt 0,2 bis 0,8, besonders bevorzugt 0,25 bis 0,7, insbesondere 0,3 bis 0,6 betragen.

Die erfindungsgemäßen Emulsionen sind insbesondere dadurch charakterisiert, daß sie zur Einstellung der Fließeigenschaften anorganische oder organische Elektrolyte enthalten. Dabei führt der Zusatz von Elektrolyten zu einer Erhöhung der Viskosität. Vorzugsweise wird der Emulsion mehr als $10^{-6}$ mol/l Elektrolyt, bevorzugt $10^{-6}$ mol/l bis 1 mol/l Elektrolyt, besonders bevorzugt $10^{-5}$ mol/l bis 0,1 mol/l Elektrolyt und ganz besonders bevorzugt $10^{-4}$ mol/l bis 0,05 mol/l Elektrolyt zugefügt. Die Zugabe des Elektrolyts kann dabei während des Emulgierprozesses oder nachträglich zur Einstellung der Viskosität erfolgen.

Die erfindungsgemäßen Emulsionen sind ferner dadurch charakterisiert, daß die Ionenstärke I der Emulsion mehr als $10^{-6}$ mol/l, bevorzugt $10^{-6}$ mol/l bis 16 mol/l, besonders bevorzugt $10^{-5}$ mol/l bis 1,6 mol/l und ganz besonders bevorzugt $10^{-4}$ mol/l bis 0,8 mol/l beträgt, wobei die Ionenstärke I definiert ist als $I = ½ * \Sigma c_i * z_i^2$, wobei $c_i$ die Konzentration des i-ten Ions und $z_i$ dessen Ladung bedeutet.

Die Elektrolytzugabe kann dabei während der Herstellung der Emulsion bzw. nachträglich in die fertige Emulsion erfolgen. Bevorzugt ist die nachträgliche Zugabe.

Bei den Elektrolyten kann es sich um niedermolekulare organische oder anorganische Verbindungen oder um hochmolekulare Oligo- bzw. Polyelektrolyte handeln.

Die erfindungsgemäß eingesetzten Elektrolyte können in Form eines Reinstoffes oder als Mischung verschiedener Elektrolyte eingesetzt werden. Bevorzugt sind Reinstoffe.

Beispiele für erfindungsgemäß einsetzbare Elektrolyte sind lösliche Halogenide, Nitrate, Nitrite, Sulfate, Sulfite, Phosphate, Phosphite von Metallen bzw. Halbmetallen der 1. bis 4. Hauptgruppe wie Lithiumchlorid, Natriumchlorid, Kaliumchlorid, Lithiumbromid, Natriumbromid, Kaliumbromid, Lithiumsulfat, Natriumsulfat, Kaliumsulfat, Lithiumnitrat, Natriumnitrat, Kaliumnitrat, Lithiumphosphat, Natriumphosphat, Kaliumphosphat, Magnesiumchlorid, Calciumchlorid, Bariumchlorid, Magnesiumsulfat, Magnesiumnitrat, Calciumnitrat, Bariumnitrat, Magnesiumphosphat, Aluminiumchlorid, Aluminiumnitrat, Aluminiumsulfat, Bleinitrat, Bleichlorid; lösliche Halogenide, Nitrate, Nitrite, Sulfate, Sulfite, Phosphate, Phosphite von Metallen der 1. bis 10. Nebengruppe wie Titantetrachlorid, Chromtrichlorid, Eisendichlorid, Eisentrichlorid, Nickelchlorid, Zinkchlorid, Zirkontetrachlorid, Zinksulfat, Zinknitrat, Eisensulfat.

Beispiele für weitere einsetzbare Elektrolyte sind die Alkali- und Erdalkalisalze von organischen Säuren wie Natriumformiat, Natriumacetat, Natriumcitrat, Natriumtartrat, Natriummethacrylat, Kaliumformiat, Kaliumacetat, Kaliumcitrat, Kaliumtartrat, Kaliummethacrylat.

Weitere Beispiele sind wasserlösliche Tetraalkylammoniumverbindungen wie Tetramethylammoniumchlorid, Tetraethylammoniumchlorid, Tetramethylammoniumbromid, Tetraethylammoniumbromid.

Beispiele für Polyelektrolyte sind Polytetraalkylammoniumverbindungen wie Polydiallydimethylammoniumchlorid, Polymethacrylate oder Polyacrylate.

Als Elektrolyte sind Alkali- und Erdalkalisalze bevorzugt, besonders bevorzugt sind Natrium- und Calcium-Salze, wobei Natrium- und Calciumchlorid ganz besonders bevorzugt sind.

Die erfindungsgemäßen Emulsionen sind insbesondere dadurch charakterisiert, daß durch Zusatz der oben beispielhaft genannten Elektrolyte die Viskosität der Emulsion ansteigt und die relative Viskosität $\eta_{rel}$ einen Wert größer 1,005 aufweist. Vorzugsweise ist $\eta_{rel} > 1,005$ bei einer Ionenstärke I der erfindungsgemäßen Emulsion im Bereich von $1 \cdot 10^{-5}$ mol/l bis 200 mol/l, bevorzugt ist $\eta_{rel} > 1,005$ bei einer Ionenstärke I der erfindungsgemäßen Emulsion im Bereich von $5 \cdot 10^{-5}$ mol/l bis 100 mol/l und ganz besonders bevorzugt ist $\eta_{rel} > 1,005$ bei einer Ionenstärke I der erfindungsgemäßen Emulsion im Bereich von $1 \cdot 10^{-4}$ mol/l bis 20 mol/l, wobei die relative Viskosität $\eta_{rel}$ definiert ist als $\eta_{rel} = \eta/\eta_0$, wobei $\eta$ die absolute Viskosität der erfindungsgemäßen Emulsion und $\eta_0$ die absolute Viskosität der Ausgangsemulsion bei einer Ionenstärke von $I \leq 1 \cdot 10^{-6}$ mol/l ist, jeweils gemessen bei einer Scherrate von 10 $s^{-1}$ und einer Temperatur

von 25°C mit einem Kegel-Platte-Meßsystem und einem Meßspalt von 105 μm.

Die erfindungsgemäßen Emulsionen sind ferner dadurch charakterisiert, daß sie bei einer Leitfähigkeit der Emulsion größer oder gleich 1 $\mu S \cdot cm^{-1}$ eine Viskosität größer als 0,001 Pas aufweisen. Vorzugsweise ist bei einer Leitfähigkeit der Emulsion im Bereich von 1 $\mu S \cdot cm^{-1}$ bis 10 $S \cdot cm^{-1}$ die Viskosität 0,05 Pas bis $1 \cdot 10^6$ Pas, bevorzugt ist bei einer Leitfähigkeit der Emulsion im Bereich von 1 $\mu S \cdot cm^{-1}$ bis 10 $S \cdot cm^{-1}$ die Viskosität 0,1 Pas bis $5 \cdot 10^5$ Pas, ganz besonders bevorzugt ist bei einer Leitfähigkeit der Emulsion im Bereich von 1 $\mu S \cdot cm^{-1}$ bis 5 $S \cdot cm^{-1}$ die Viskosität 0,1 Pas bis $1 \cdot 10^5$ Pas und in einer speziellen Ausführung ist bei einer Leitfähigkeit der Emulsion im Bereich von 1 $\mu S \cdot cm^{-1}$ bis 0,1 $S \cdot cm^{-1}$ die Viskosität 0,1 Pas bis $1 \cdot 10^5$ Pas wobei die Viskosität gemessen wird bei einer Scherrate von 10 $s^{-1}$ und einer Temperatur von 25 °C mit einem Kegel-Platte-Meßsystem und einem Meßspalt von 105 μm und die Leitfähigkeit der Emulsion sich ergibt gemäß Leitfähigkeit der Emulsion = gemessene Leitfähigkeit / Volumenbruch der Wasserphase und die Leitfähigkeit der Emulsion gemessen wird mittels einer Leitfähikeitsmeßzelle QCond 2400.

Die Emulsionen sind ferner dadurch charakterisiert, daß beim erfindungsgemäßen Zusatz von Elektrolyt der Verlustfaktor tan δ einen Wert kleiner 1 aufweist. Vorzugsweise ist $\tan \delta < 1$ bei einer Ionenstärke I im Bereich von $1 \cdot 10^{-5}$ mol/l bis 200 mol/l, bevorzugt ist $\tan \delta < 1$ bei einer Ionenstärke I im Bereich von $5 \cdot 10^{-5}$ mol/l bis 100 mol/l und ganz besonders bevorzugt ist $\tan \delta < 1$ bei einer Ionenstärke I im Bereich von $1 \cdot 10^{-4}$ mol/l bis 20 mol/l, wobei tan δ definiert ist als der Quotient G''/G' des Verlustmoduls G'' und des Speichermoduls G' und tan δ erhalten wurde als Anfangsplateauwert im linear-viskoelatischen Bereich bestimmt durch ein Oszillationsexperiment unter Variation der Schubspannungsamplitude von 0,05 Pa bis 10 Pa bei einer konstanten Winkelgeschwindigkeit ω = 6,28 rad/s mit einem Kegel-Platte-Sensor-System mit einem Meßspalt von 105 μm bei einer Temperatur von 25°C und einem Volumenbruch $\Phi_w$ der Wasserphase von 0,5.

Die bedeutet weiter, daß die erfindungsgemäßen Emulsionen bei einer Leitfähigkeit der Emulsion von größer oder gleich 1 $\mu S \cdot cm^{-1}$ einen Verlustfaktor tan δ kleiner 1 aufweisen. Bevorzugt ist $\tan \delta < 1$ bei einer Leitfähigkeit der Emulsion im Bereich von 5 $\mu S \cdot cm^{-1}$ bis 10 $S \cdot cm^{-1}$, besonders bevorzugt ist $\tan \delta < 1$ bei einer Leitfähigkeit der Emulsion im Bereich von 10 $\mu S \cdot cm^{-1}$ bis 0,1 $S \cdot cm^{-1}$, wobei tan δ definiert ist als der Quotient G''/G' des Verlustmoduls G'' und des Speichermoduls G' und tan δ erhalten wurde als Anfangsplateauwert im linear-viskoelatischen Bereich bestimmt durch ein Oszillationsexperiment unter Variation der Schubspannungsamplitude von 0,05 Pa bis 10 Pa bei einer konstanten Winkelgeschwindigkeit ω = 6,28 rad/s mit einem Kegel-Platte-Sensor-System mit einem Meßspalt von 105 μm bei einer Temperatur von 25 °C und einem volumenbruch $\Phi_w$ der Wasserphase von 0,5 und die Leitfähigkeit der Emulsion sich ergibt gemäß Leitfähigkeit der Emulsion = gemessene Leitfähigkeit / Volumenbruch der Ölphase und die Leitfähigkeit der Emulsion gemessen wird mittels einer Leitfähikeitsmeßzelle QCond 2400.

Die erfindungsgemäßen Emulsionen sind ferner dadurch charakterisiert, dass die mittlere Partikelgröße der dispersen Phase, d.h. der mittlere Tropfendurchmesser, gemessen mittels Laserbeugung, z.B. auf einem Laserbeugungsgerät der Fa. Sympatec in Küvettenmeßtechnik, vorzugsweise 0,5 μm bis 500 μm, bevorzugt 0,7 μm bis 100 μm, besonders bevorzugt 0,7 μm bis 50 μm und ganz besonders bevorzugt 0,7 pm bis 20 pm beträgt.

Ein weiterer Gegenstand ist ein Verfahren zur Herstellung der Emulsionen, wobei in einem ersten Schritt eine hochkonzentrierte feinteilige Dispersion der entsprechenden Kieselsäure in der Flüssigkeit, die in der Emulsion die homogene Phase bildet hergestellt wird und in einem zweiten Schritt eine hochviskose Präemulsion hergestellt wird, welche die Gesamtmenge der dispersen Phase und die im ersten Schritt hergestellte hochkonzentrierte feinteilige Dispersion der Kieselsäure in der Flüssigkeit, die in der erfindungsgemäßen Emulsion die homogene Phase bildet aufweist, wobei das eingesetzte Volumen der Dispersion so bemessen ist, dass die gesamte Menge der benötigen sinteraggregierten Kieselsäure enthalten ist, und in einem dritten Schritt die restliche homogenen Phase langsam eindosiert wird und in einem beliebigen der Schritte ein Elektrolyt eindosiert wird.

Als wesentlich für das Erzielen der oben beschriebenen Eigenschaften der erfindungsgemäßen Emulsionen erwies sich dabei ein Emulgierverfahren, bei dem während der Emulgierung ein Zustand hoher Viskosität, im weiteren als 'steife Phase' bezeichnet, durchlaufen wird.

Im einzelnen weist der Prozess zur Herstellung der erfindungsgemäßen Emulsionen folgende Einzelschritten auf:

- Herstellung einer hochkonzentrierten feinteiligen Dispersion der entsprechenden geeigneten Kieselsäure in der Flüssigkeit, die in der erfindungsgemäßen Emulsion die homogene Phase bildet.
- Herstellung einer hochviskosen Präemulsion, bestehend aus der hochkonzentrierten feinteiligen Dispersion der entsprechenden geeigneten Kieselsäure in der Flüssigkeit, die in der erfindungsgemäßen Emulsion die homogene Phase bildet, wobei das eingesetzte Volumen so bemessen ist, dass die gesamte Menge der benötigen sinteraggregierten Kieselsäure enthalten ist, und der Gesamtmenge der dispersen Phase
- langsames Eindosieren der restlichen homogenen Phase unter Scheren

Die Herstellung der hochkonzentrierten feindispersen Dispersion der entsprechenden geeigneten Kieselsäure in der Flüssigkeit, die in der erfindungsgemäßen Emulsion die homogene Phase bildet, kann dabei grundsätzlich gemäß

den bekannten Verfahren zur Herstellung von Kieselsäuredispersionen erfolgen, wie das Einarbeiten mittels Rührorgane mit hoher Scherwirkung wie schnellaufende Rührer, schnellaufende Dissolver, Rotor-Stator-Systeme, Ultraschalldispergatoren oder Kugel- bzw. Perlmühlen.

Die Konzentration der Kieselsäure-Sinteraggregate in der Dispersion beträgt dabei zwischen 1 und 80 Gew. %, bevorzugt zwischen 10 und 60 Gew.%, besonders bevorzugt zwischen 10 und 40 Gew.% und ganz besonders bevorzugt zwischen 12 und 30 Gew.%.

Die Herstellung der hochviskosen Präemulsion kann grundsätzlich gemäß den bekannten Verfahren zur Herstellung von Emulsionen erfolgen, es zeigte sich jedoch, dass die im folgenden beschriebenen Verfahren besonders geeignet sind, die erfindungsgemäßen Emulsionen zu erhalten.

Verfahren 1:

- Vorlegen der oben beschriebenen hochkonzentrierten Kieselsäuredispersion, wobei das vorgelegte Volumen so bemessen ist, dass es die Gesamtmenge an benötigen Kieselsäuresinteraggregaten enthält.
- Langsames Zudosieren des Gesamtvolumens an disperser Phase unter ständigem Homogenisieren z.B. mittels eines schnellaufenden Rührers, schnellaufenden Dissolvers oder eines Rotor-Stator-Systems.
- Anschließend langsames Zudosieren des gewünschten Restvolumens an reiner homogener Phase gegebenenfalls unter ständigem Homogenisieren z.B. mittels eines schnellaufenden Rührers, schnellaufenden Dissolvers oder eines Rotor-Stator-Systems.

Verfahren 2:

- Vorlegen des Gesamtvolumens an disperser Phase.
- Langsames Zudosieren der oben beschriebenen hochkonzentrierten Kieselsäuredispersion unter ständigem Homogenisieren z.B. mittels eines schnellaufenden Rührers, schnellaufenden Dissolvers oder eines Rotor-Stator-Systems, wobei das zudosierte Volumen so bemessen ist, dass es die Gesamtmenge an benötigen Kieselsäuresinteraggregaten enthält.
- Anschließend langsames Zudosieren des gewünschten Restvolumens an reiner homogener Phase gegebenenfalls unter ständigem Homogenisieren z.B. mittels eines schnellaufenden Rührers, schnellaufenden Dissolvers oder eines Rotor-Stator-Systems

Die beschriebenen Verfahren können sowohl in kontinuierlicher als auch in diskontinuierlicher Form durchgeführt werden. Bevorzugt ist die kontinuierliche Form.

Die Temperatur der flüssigen Phase während des Emulgierprozesses liegt zwischen 0 °C und 80 °C, bevorzugt zwischen 10 °C und 50 °C, besonders bevorzugt zwischen 20 °C und 40 °C.

Der Emulgierprozess kann bei Normaldruck, also bei 900 bis 1100 hPa, bei erhöhtem Druck oder im Vakuum durchgeführt werden. Bevorzugt ist der Prozess bei Normaldruck.

Die erfindungsgemäßen Emulsionen können für alle Zwecke eingesetzt werden, für die bereits bisher Emulsionen verwendet werden. Insbesondere sind dies wasserbasierende Beschichtungs-, Kleb- und Dichtstoffe, enthaltend z.B. Organosiliciumverbindungen, wie Organo(poly)silane, Organo(poly)siloxane, Organo(poly)silazane und Organo (poly)silcarbane; Polyolefine, wie silylterminierte Polyisobutylene (z.B. erhältlich unter der Marke Epion von Kaneka Corp., Japan); Polyurethane, Polyole, wie Hydroxy-haltige Polyester, Hydroxy-haltige Polyether, Methyldimethoxysilylpropyl terminierte Polypropylenglycole (z.B. erhältlich als "MS-Polymere" von der Fa. Kaneka Corp. Japan), Hydroxyhaltige Polyacrylate; Polyisocyanate, wie aliphatische und aromatische Polyisocyanate, Isocyanat terminierte Polyurethan-Präpolymere, hergestellt durch Umsetzung von Polyolen mit Polyisocyanaten im Überschuss, sowie deren silyterminierte Derivate (z.B. erhältlich unter der Bezeichnung DESMOSEAL® von der Bayer AG, Deutschland; (Poly) Epoxyverbindungen, wie Bisphenol A basierende Epoxide, monomere, oligomere und polymere Glycidoxy-Funktionen enthaltende Verbindungen, wie Diglycidylether basierend auf Bisphenol A, Epoxy-Novolac-Grundstoffe und Harze, Epoxyalkydharze, Epoxyacrylate, aliphatische Epoxide wie lineare Alkylenbisglycidylether und cycloaliphatische Glycidylether, wie 3,4-Epoxycyclohexyl-3,4-Epoxy-Cyclohexane-Carboxylate und aromatische Epoxide, wie Triglycidylether von p-Aminophenol und Triglycidylether von Methylendianilin; (Poly)Amine, wie cyclische und lineare Amine, wie Hexamethylendiamin, aromatische Amine, wie 4,4-Methylen-bis(2,6-diethylanilin), Bis-(2-aminoalkyl)-Polyalkylenoxid, wie Bis-(2-aminopropyl)-Polypropylengly-col und Jeffamine, (Poly)Amidoamine, (Poly)Mercaptane, (Poly)-Carbonsäure, (Poly)Carbonsäureanhydride; Acrylate und deren Ester, wie Glycidylacrylate, Alkylacrylate und deren Ester, Methacrylate

und deren Ester, Polysulphide bildende Polymere und Polysulfide, wie Thioplaste (z.B. erhältlich unter der Marke Thiokol der Fa. Toray Thiokol Co. Ltd.).

Beispiele für Epoxyverbindungen sind Alkylenbisglycidylether, wie

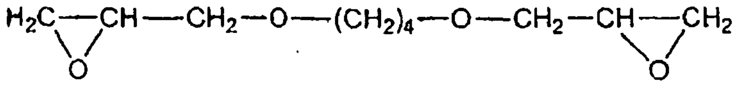

Bisphenol A basierende Diglycidylether, wie

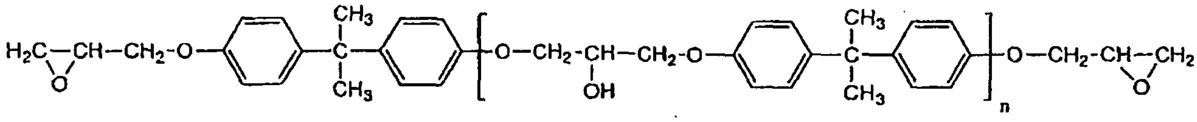

mit n bevorzugt von 0 bis 10, besonders bevorzugt 0 bis 5.

Beispiele für Epoxy-Novolac-Harze sind solche der Formel

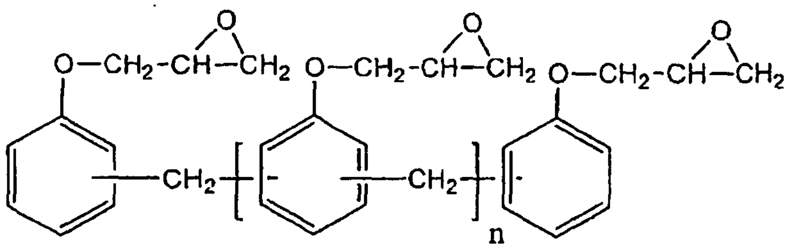

bifunktionelle Epoxyverbindungen, wie

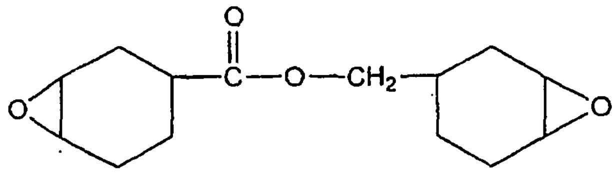

trifunktionelle Epoxyverbindungen, wie

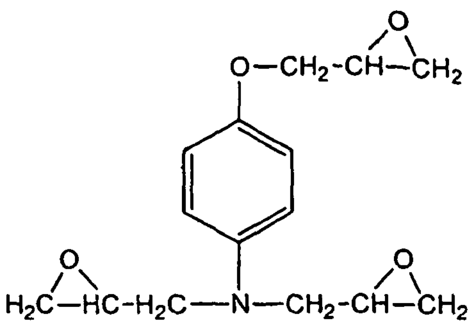

tetrafunktionelle Epoxyverbindungen, wie

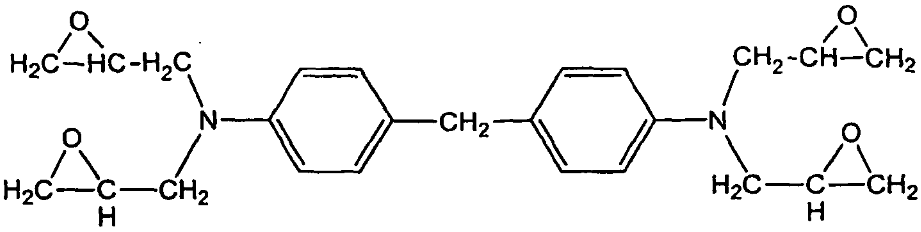

Ferner können die erfindungsgemäßen Emulsionen eingesetzt werden für kosmetische und pharmazeutische Anwendungen, Putz- und Reinigungsmittel oder Anwendungen zur Veränderung der Grenzflächeneigenschaften von festen und flüssigen Substraten, wie z.B. Hydrophobiermittel, Haftvermittlern, Trennmittel, Papierbeschichtungen oder Schaumkontrollmittel.
Ferner können die erfindungsgemäßen Emulsionen zur Herstellung von w/o/w- bzw. o/w/o-Mehrfachemulsionen verwendet werden, beispielsweise als Controll-Release-Systeme oder zur Segregation von Reaktivstoffen.

**Beispiel 1:**

Herstellung von Feststoffpartikeln

100 g einer pyrogenen Kieselsäure mit einer spezifischen BET-Oberfläche, gemessen nach DIN 66131 und DIN 66132, von 200 $m^2/g$ (erhältlich bei Wacker-Chemie GmbH, D-München unter dem Namen Wacker HDK® N20) werden unter Rühren (mit 1000 UpM bei einem Rührflügeldurchmesser von 12,5 cm) fluidisiert, dann während 15 Minuten mit Stickstoffgas beaufschlagt und inertisiert, anschließend der Stickstoffstrom wieder abgestellt. Dann werden 2 g Dimethyldichlorsilan mit einer Zweistoffdüse als Aerosol in die fluidisierte Kieselsäure aufgesprüht, bei einer Temperatur von ca. 25°C und einem Umgebungsdruck von ca. 1013 hPa. Nach 30 Minuten weiterem Rühren wird anschließend die so behandelte Kieselsäure für 2 Stunden bei 300°C in einem Ofen von 100 1 Rauminhalt getempert, unter leichtem Stickstoffstrom von 1000 1/h.
Erhalten wird eine weiße pulverförmige Kieselsäure mit folgenden Eigenschaften:
- Die Kieselsäure ist bedingt, aber nicht vollständig Wasser-benetzbar; dies zeigt sich dadurch, dass sich von der Kieselsäure nur 18 Gew.-% in Wasser mit einem Ultraturrax zu einer für einen Tag stabilen fließfähigen Masse einarbeiten lassen, unter den gleichen Bedingungen und zu gleicher Viskosität aber 24 Gew.-% der Ausgangskieselsäure (Wacker HDK® N20), die vollständig wasserbenetzbar ist.
- Weitere Eigenschaften der Kieselsäure sind in Tabelle 1 zusammengefaßt.

Tabelle 1:

| Eigenschaft | Kieselsäure B1 nach Beispiel 1 |
|---|---|
| BET-Oberfläche | 184 $m^2/g$ |
| Restgehalt an nicht silylierten Kieselsäure-Silanolgruppen | 80% |
| Kohlenstoffgehalt %C | 0,5 Gew.-% |
| Methanolzahl | 0 |
| Kontaktwinkel THETA Methode-1 gegen Wasser und Luft | 84° |
| Kontaktwinkel THETA Methode-2 gegen Wasser und Luft | von 80° |
| Oberflächenenergie GAMMA | 69 $mJ/m^2$ |
| Dispersionsanteil der Oberflächenenergie GAMMA-s-D | 65 $mJ/^2$ |

- Spezifische BET-Oberfläche, gemessen nach DIN 66131 und DIN 66132

- Restgehalt an nicht silylierten Kieselsäure-Silanolgruppen, erhalten als Quotient (a) der Menge der Kieselsäuresilanolgruppen, der wie o.g. hergestellten Kieselsäure dividiert durch die Menge der Kieselsäuresilanolgruppen der unbehandelten Ausgangskieselsäure (Wacker HDK® N20); die Menge der Kieselsäuresilanolgruppen wird durch Säure-Base-Titration bestimmt (analog G.W. Sears Anal. Chem, 28 (12), (1950), 1981). Methode: Säure-Base-Titration der in Wasser/Methanol = 50:50 supendierten Kieselsäure; Titration im Bereich oberhalb des pH-Bereichs des isoelektrischen Punktes und unterhalb des pH-Bereichs der Auflösung der Kieselsäure; unbehandelte Kieselsäure mit 100% SiOH (Kieselsäure-Oberflächensilanolgruppen): SiOHphil = 1.8 SiOH / $nm^2$; silylierte Kieselsäure: SiOH-silyl; Restgehalt an nicht silylierten KieselsäureSilanolgruppen: %SiOH = SiOH-silyl/SiOH-phil*100%
- Kohlenstoffgehalt %C bestimmt mittels Elementaranalyse auf Kohlenstoff; Verbrennen der Probe bei über 1000°C im $O_2$-Strom, Detektion und Quantifizierung des entstehenden $CO_2$ mit IR; Gerät LECO 244
- Methanolzahl, gemessen wie folgt: Test (Volumen% MeOH in Wasser) der Benetzbarkeit mit Masser-Methanol Gemischen = Methanolzahl (MZ): Einschütteln eines gleichen Volumens der Kieselsäure mit gleichem Volumen an Wasser-Methanol Gemisch; Start mit 0% Methanol; bei Nicht-Benetzung schwimmt Kieselsäure auf: Es ist ein Gemisch mit um 5 Vol% höherem MeOH Gehalt zu verwenden; bei Benetzung sinkt Kieselsäure ein: Anteil MeOH (%) in Wasser gibt Methanolzahl (MZ)
- Kontaktwinkel THETA Methode-1 gegen Wasser, gemessen wie folgt: Der Kontaktwinkel der Partikel wird durch sorgfältiges Herstellen, mit üblichen Methoden, eines Presslings der Kieselsäure und anschließender Bestimmung des Kontaktwinkels gegen Wasser, hier ein aufliegender Wassertropfen aus bidestilliertem Wasser an Luft durch digitale Bildauswertung erhalten.

Der Kontaktwinkel θ definiert das Verhältnis der Oberflächenspannungen und -energien γ von Flüssigkeiten (1) und Feststoffen (s) in einem Gasraum (g) wie folgt.

$$\cos(\theta) = (\gamma(sl) - \gamma(sg)) / \gamma(lg)$$

Die Oberflächenenergie ($mJ/m^2$) eines Feststoffes ist dimensionsgleich mit der Oberflächenspannung einer Flüssigkeit (mN/m), da gilt (J) = [N*km].

- Kontaktwinkel THETA Methode-2 gegen Wasser, gemessen wie mittels Imbibitionsmethode unter Verwendung der Lucas-Washburn-Gleichung, beruhend auf dem Einsaugen von bekannten und definierten Flüssigkeit, mit bekannter Oberflächenspannung, in ein definiertes Haufwerk, wie hier ein schwach verdichteter Pressling aus der Kieselsäure mit offener Porosität größer 0,25 und Porenradius r. Die Aufsauggeschwindigkeit dh/dt, bzw. die Höhe der aufgesaugten Flüssigkeitsäule h, berechnet aus der Massenaufnahme m an Flüssigkeit durch das Partikelhaufwerk gegen die Zeit t, sowie die Viskosität der aufgesaugten Flüssigkeit η sowie die Oberflächenspannung γ der aufgesaugten Flüssigkeit lassen bei bekanntem Partikelradius r mittels der Gleichung nach Lucas-Washburn (Washburn, E.W., Phys. Rev. 17, 273 (1921) und R. Lucas Kolloid Z. 23, 15 (1918) den Wert Cosinus von θ (cos (θ) ) und damit den Kontakt- oder Randwinkel θ der Flüssigkeit gegen die Partikeloberfläche ermitteln; folgend J. Schoelkopf et al, J. Colloid. Interf. Sci. 227, 119-131 (2000)

Als Flüssigkeit mit bekannter Oberflächenspannung werden Methanol-Wasser-Mischungen der Mischungsverhältnisse (Volumen Methanol zu Volumen Wasser) 0:100, 5:95, 10:90, 15:85, 20:80, 25:75, 30:70, 35:65, 40:60, 45: 55, 50:50, 55:45, 60:40, 65:35, 70:30, 75:25, 80:20, 85:15, 90:10, 95:5, 100:0 verwendet.

$$dh/dt = r * \gamma * \cos(\theta) / (4 * \eta)$$

und

$$h^2 = r * \gamma * t * \cos(\theta) / (2 * \eta)$$

| | |
|---|---|
| $t = A \cdot m^2$ | Washbum-Gleichung |
| mit t : | Zeit |
| m : | Masse der angesaugten Flüssigkeit |

$$A = \frac{\eta}{\{C \cdot \rho^2 \cdot \gamma \cdot \cos \vartheta\}}$$

$\eta$ : Viskosität der Flüssigkeit
$\rho$ : Dichte der Flüssigkeit
$\gamma$ : Oberflächenspannung der Flüssigkeit
$\vartheta$ : Randwinkel Flüssigkeit-Pulver
C : Faktor, nur abhängig von den geometrischen Eigenschaften des Pulvers und Probenrohrs

Eine Illustration des Messverfahrens ist in Figur 5 zu finden.

- Die Oberflächenenergie GAMMA kann für Partikel als kritische Oberflächenenergie GAMMA-krit mittels eines Zisman-Plots bestimmt werden, der den jeweiligen Kontaktwinkel THETA der Kieselsäure gegen eine definierte Flüssigkeit, wie oben mit der Imbibitionsmethode bestimmt, gegen die Kontaktwinkel der jeweiligen Flüssigkeiten aufträgt.

- Für Partikel wie pyrogene Kieselsäure, die Agglomerate mit Schüttdichten $d_{SD}$ << 1 g/ml bildend, aber bestehend aus Primärpartikeln mit Materialdichten $d_{MD}$ > 1 g/ml, kann Einschütteln in Flüssigkeiten verschiedener Oberflächenspannung als Methode herangezogen werden: Bei Nichtbenetzung schwimmen die Partikel-Agglomerate auf; bei Benetzung wird die Luft in den Agglomeraten verdrängt, und die Partikel-Agglomerate sinken ein.
Bei Verwendung verschiedener Flüssigkeiten mit verschiedener Oberflächenspannung kann exakt die Oberflächenspannung einer Flüssigkeit ermittelt werden, bei der die Partikel-Agglomerate einsinken; diese liefert die kritische Oberflächenenergie $\gamma_{crit}$ als Maß für die Oberflächenenergie $\gamma$ der Partikel.
Die Methode kann auch dergestalt vereinfacht werden, dass die Oberflächenspannung von Wasser (72,5 mN/m) durch Zugabe von Methanol, Ethanol oder iso-Propanol verringert wird.
Typischerweise kann Wasser vorgelegt werden, eine bestimmte Menge an Partikel-Agglomeraten auf die Wasseroberfläche aufgelegt (schwimmend) und dann der Alkohol, unter Rühren, zutitriert werden. Das Wasser zu Alkohol-Verhältnis bei Einsinken der Partikel-Agglomerate wird notiert und genau für dieses Verhältnis Wasser : Alkohol in einem getrennten Versuch mit Standardmethoden (Ringabreißmethode, Wilhelmy-Methode) die Oberflächenspannung bestimmt.
Effektiver, und wie hier durchgeführt, werden definierte Mischungen von Wasser mit Methanol hergestellt, und dann werden die Oberflächenspannungen dieser Gemische bestimmt. In einem getrennten Experiment werden diese Wasser : Methanol Mischungen mit definierten Mengen an Partikel-Agglomeraten überschichtet (beispielsweise in einem Volumenverhältnis 1:1) und unter definierten Bedingungen geschüttelt (beispielweise schwaches Schütteln mit der Hand oder mit einem Taumelmischer für ca. 1 Minute).
Bestimmt wird das Wasser : Methanol-Gemisch, bei dem die Partikel-Agglomerate eben noch nicht einsinken und das Wasser : Methanol-Gemisch mit höherem Methanolgehalt, bei dem die Partikel-Agglomerate eben einsinken. Die Oberflächenspannung des letzteren Methanol : WasserGemisches liefert die kritische Oberflächenenergie $\gamma_{crit}$ als Maß für die Oberflächenenergie $\gamma$ der Partikel, wie in Tabelle 1 gegeben.

- Der Dispersions-Anteil der Oberflächenenergie gamma-s-D wird mit der Inversen Gaschromatographie und Alkanen als Sonden bestimmt, folgend "Inverse Gaschromatographie" - "Characterisation of Polymers and other Materials", 391 ACS Symposium Series, D R Lloyd, Th C Ward, H P Schreiber, Chapter 18, pp 248-261, ACS, Washington DC 1989, ISBN 0-8412-1610-X.

Herstellung einer wäßrigen Dispersion (nicht erfindungsgemäß)

In einem 500 ml Edelstahlbecher werden mittels eines Dissolver mit Sthlagzahnscheibe 18 g der oben beschriebenen teilhydrophoben Kieselsäure in 82 g vollentsalztem (VE)-Wasser vor dispergiert. Die erhaltene hochviskose aber noch fließfähige Masse wird durch eine Ultraschallzelle mit einer Durchflußrate von 10 ml pro Minute und einer Amplitudenleistung von 300 Watt gepumpt. Die analytischen Daten der so erhaltenen Dispersion sind in Tabelle 2 zusammengefasst. Tabelle 2:

| Eigenschaft | wäßrige Dispersion aus Beispiel 1 |
|---|---|
| Feststoffgehalt | 18,0% |

(fortgesetzt)

| Eigenschaft | wäßrige Dispersion aus Beispiel 1 |
|---|---|
| pH-Wert | 5,2 |
| mittlerer Durchmesser Sinteraggregate | 311 nm |
| Viskosität | 280 mPas |

- Feststoffgehalt der Dispersion bestimmt nach folgender Methode: 10 g wässrige Dispersion werden in einer Porzellanschale mit der gleichen Menge Ethanol versetzt und in einem $N_2$-gespülten Trockenschrank bei 150°C zur Gewichtskonstanz eingedampft. Die Masse $m_s$ des trockenen Rückstandes ergibt den Feststoffgehalt gemäß Feststoffgehalt / % = $m_s$ * 100 / 10 g.
- pH-Wert gemessen mittels pH-Einstabmesskette
- mittlerer Durchmesser der Sinteraggregate gemessen mittels Photokorrelationsspektroskopie nach folgender Methode: Von der zu messenden Dispersion werden 4 Proben mit einem Kieselsäuregehalt von 1 Gew.%, 0,75 Gew. %, 0,5 Gew.% und 0,25 Gew.% durch Einrühren der entsprechenden Menge Ausgangsdispersion mittels Magnetrührer in VE-Wasser hergestellt. Die Proben werden in einem PCS-Gerät Coulter N4 Plus der Fa. Coulter bei Detektionswinkeln von 30,1°, 62,6° und 90° vermessen. Der mittlere Durchmesser der Sinteraggregate ergibt sich aus einer Extrapolation der erhaltenen winkelabhängigen Meßwerte auf einen Kieselsäuregehalt von 0 Gew.% und anschließende Mittelung über die drei gemessenen Winkel.
- Viskosität der Dispersion bestimmt mit einem Rheometer MCR 600 der Fa. Haake mit Kegel-Platte-Sensorsytem (105 μm Meßspalt) bei 25°C und einer Scherrate D = 10 $s^{-1}$.

**Vergleichsbeispiel: Herstellung einer Emulsion (nicht erfindungsgemäß)**

69,5 g der oben beschriebenen Kieselsäuredispersion mit einem Feststoffgehalt von 18 Gew.% werden in einem 500 ml Edelstahlbecher vorgelegt. Unter Rühren bei 10000 rpm mit einem Ultraturrax und Wasserkühlung werden über einem Zeitraum von 15 min langsam 150 g eines Polydimethylsiloxans mit einer Viskosität von 100 mPas (erhältlich unter dem Namen "AK100" bei Wacker-Chemie GmbH, D-München) zudosiert. Dabei sollte die Temperatur der Mischung nicht über 60 °C steigen. Zu dieser nun hochviskosen standfesten Masse werden anschließend 93 g VE-Wasser ebenfalls bei 10000 rpm langsam über einen Zeitraum von 15 min zugegeben. Dabei sollte die Temperatur der Mischung nicht über 60 °C steigen. Es resultiert eine dünnflüssige weiße O/W-Emulsion, deren analytische Daten in Tabelle 3 zusammengefaßt sind.

**Beispiel 2 (erfindungsgemäß)**

69,5 g einer gemäß Beispiel 1 erhaltenen Kieselsäuredispersion mit einem Feststoffgehalt von 18 Gew.% werden in einem 500 ml Edelstahlbecher vorgelegt. Unter Rühren bei 10000 rpm mit einem Ultraturrax und Wasserkühlung werden über einem Zeitraum von 15 min langsam 150 g eines Polydimethylsiloxans mit einer Viskosität von 100 mPas (erhältlich unter dem Namen "AK100" bei Wacker-Chemie GmbH, D-München) zudosiert. Dabei sollte die Temperatur der Mischung nicht über 60 °C steigen. Zu dieser nun hochviskosen standfesten Masse werden anschließend 93 g VE-Wasser ebenfalls bei 10000 rpm langsam über einen Zeitraum von 15 min zugegeben. Dabei sollte die Temperatur der Mischung nicht über 60°C steigen. Anschließend werden der Emulsion unter Rühren 0,0035 g NaCl zugesetzt. Es resultiert eine weiße O/W-Emulsion, deren analytische Daten in Tabelle 3 zusammengefaßt sind.

**Beispiel 3 (erfindungsgemäß)**

69,5 g einer gemäß Beispiel 1 erhaltenen Kieselsäuredispersion mit einem Feststoffgehalt von 18 Gew.% werden in einem 500 ml Edelstahlbecher vorgelegt. Unter Rühren bei 10000 rpm mit einem Ultraturrax und Wasserkühlung werden über einem Zeitraum von 15 min langsam 150 g eines Polydimethylsiloxans mit einer Viskosität von 100 mPas (erhältlich unter dem Namen "AK100" bei Wacker-Chemie GmbH, D-München) zudosiert. Dabei sollte die Temperatur der Mischung nicht über 60°C steigen. Zu dieser nun hochviskosen standfesten Masse werden anschließend 93 g VE-Wasser ebenfalls bei 10000 rpm langsam über einen Zeitraum von 15 min zugegeben. Dabei sollte die Temperatur der Mischung nicht über 60°C steigen. Anschließend werden der Emulsion unter Rühren 0,022 g NaCl zugesetzt. Es resultiert eine weiße O/W-Emulsion, deren analytische Daten in Tabelle 3 zusammengefaßt sind.

**Beispiel 4 (erfindungsgemäß)**

69,5 g einer gemäß Beispiel 1 erhaltenen Kieselsäuredispersion mit einem Feststoffgehalt von 18 Gew.% werden in einem 500 ml Edelstahlbecher vorgelegt. Unter Rühren bei 10000 rpm mit einem Ultraturrax und Wasserkühlung werden über einem Zeitraum von 15 min langsam 150 g eines Polydimethylsiloxans mit einer Viskosität von 350 mPas (erhältlich unter dem Namen "AK350" bei Wacker-Chemie GmbH, D-München) zudosiert. Dabei sollte die Temperatur der Mischung nicht über 60°C steigen. Zu dieser nun hochviskosen standfesten Masse werden anschließend 93 g VE-Wasser ebenfalls bei 10000 rpm langsam über einen Zeitraum von 15 min zugegeben. Dabei sollte die Temperatur der Mischung nicht über 60°C steigen. Anschließend werden der Emulsion unter Rühren 0,088 g NaCl zugesetzt. Es resultiert eine weiße O/W-Emulsion, deren analytische Daten in Tabelle 3 zusammengefaßt sind.

**Beispiel 5 (erfindungsgemäß)**

69,5 g einer gemäß Beispiel 1 erhaltenen Kieselsäuredispersion mit einem Feststoffgehalt von 18 Gew.% werden in einem 500 ml Edelstahlbecher vorgelegt. Unter Rühren bei 10000 rpm mit einem Ultraturrax und Wasserkühlung werden über einem Zeitraum von 15 min langsam 150 g eines Bisphenol A basierenden Epoxy-Harzes mit einer durchschnittlichen Molmasse < 700 g/mol (erhältlich unter dem Namen "Araldite GY 776 BD" bei Vantico GmbH & Co KG) zudosiert. Dabei sollte die Temperatur der Mischung nicht über 60 °C steigen. Zu dieser nun hochviskosen standfesten Masse werden anschließend 93 g VE-Wasser ebenfalls bei 10000 rpm langsam über einen Zeitraum von 15 min zugegeben. Dabei sollte die Temperatur der Mischung nicht über 60 °C steigen. Anschließend werden der Emulsion unter Rühren 0,088 g NaCl zugesetzt. Es resultiert eine weiße O/W-Emulsion, deren analytische Daten in Tabelle 3 zusammengefaßt sind.

**Beispiel 6 (erfindungsgemäß)**

69,5 g einer gemäß Beispiel 1 erhaltenen Kieselsäuredispersion mit einem Feststoffgehalt von 18 Gew.% werden in einem 500 ml Edelstahlbecher vorgelegt. Unter Rühren bei 10000 rpm mit einem Ultraturrax und Wasserkühlung werden über einem Zeitraum von 15 min langsam 150 g eines Polydimethylsiloxans mit einer Viskosität von 100 mPas (erhältlich unter dem Namen "AK100" bei Wacker-Chemie GmbH, D-München) zudosiert. Dabei sollte die Temperatur der Mischung nicht über 60°C steigen. Zu dieser nun hochviskosen standfesten Masse werden anschließend 93 g VE-Wasser ebenfalls bei 10000 rpm langsam über einen Zeitraum von 15 min zugegeben. Dabei sollte die Temperatur der Mischung nicht über 60 °C steigen. Anschließend werden der Emulsion unter Rühren 0,0416 g $CaCl_2$ zugesetzt. Es resultiert eine weiße O/W-Emulsion, deren analytische Daten in Tabelle 3 zusammengefaßt sind.

Tabelle 3

| | Vergleichsbeispiel | Beispiel 2 | Beispiel 3 | Beispiel 4 | Beispiel 5 | Beispiel 6 |
|---|---|---|---|---|---|---|
| mmol/l Elektrolyt | 0 | 0,4 | 2,5 | 10 | 10 | 1 |
| Ionenstärke/mol/l | $< 10^{-6}$ | $4{,}0\cdot10^{-4}$ | $2{,}5\cdot10^{-3}$ | 0,01 | 0,01 | $3{,}0\cdot10^{-3}$ |
| rel. Vikosität $\eta_{rel}$ | 1 | 1,22 | 2,59 | 3,82 | 4,06 | 1,94 |
| tan $\delta$ | 2,0 | 0,29 | 0,17 | 0,13 | 0,15 | 0,20 |
| Gleitfähigkeit $\mu Scm^{-1}$ | 0 | 44 | 230 | 710 | 630 | 189 |
| Viskosität Pas | 0,129 | 0,158 | 0,335 | 0,465 | 0,427 | 0,361 |
| | | | | | | |

- Leitfähigkeit gemessen mittels Leifähigkeitsmesszelle QCond 2400; Korrektur des Meßwertes auf $\Phi_w = 1$ gemäß $\Omega_{corr} = \Omega_{mess} / \Omega_w$
- Viskosität der Dispersion bestimmt mit einem Rheometer MCR 600 der Fa. Haake mit Kegel-Platte-Sensorsytem (105 pm Meßspalt) bei 25 °C und einer Scherrate D = 10 $s^{-1}$
- Verlustfaktor tan $\delta$ der Dispersion bestimmt mit einem Rheometer MCR 600 der Fa. Haake mit Kegel-Platte-Sensorsytem (105 $\mu$m Meßspalt) bei 25 °C durch ein Oszillationsexperiment unter Variation der Schubspannungsamplitude von 0,05 Pa bis 10 Pa bei einer konstanten Winkelgeschwindigkeit $\omega$ = 6,28 rad/s bei einem Volumenbruch $\Phi_w$ der Wasserphase von 0,5
- die relative Viskosität $\eta_{rel}$ ist definiert als $\eta_{rel} = \eta/\eta_0$, wobei $\eta$ die absolute Viskosität der erfindungsgemäßen Emulsion und $\eta_0$ die absolute Viskosität der Ausgangsemulsion bei einer Ionenstärke von $I \leq 1 \cdot 10^{-6}$ mol/l ist

## Patentansprüche

1. Emulsionen erhältlich durch ein Verfahren, wobei in einem ersten Schritt eine hochkonzentrierte feinteilige Dispersion der entsprechenden Kieselsäure in der Flüssigkeit, die in der Emulsion die homogene Phase bildet hergestellt wird und in einem zweiten Schritt eine hochviskose Präemulsion hergestellt wird, welche die Gesamtmenge der dispersen Phase und die im ersten Schritt hergestellte hochkonzentrierte feinteilige Dispersion der Kieselsäure in der Flüssigkeit, die in der erfindungsgemäßen Emulsion die homogene Phase bildet aufweist, wobei das eingesetzte Volumen der Dispersion so bemessen ist, dass die gesamte Menge der benötigen sinteraggregierten Kieselsäure enthalten ist, und in einem dritten Schritt die restliche homogenen Phase langsam eindosiert wird und in einem beliebigen Schritt der Schritte ein Elektrolyt eindosiert wird.

2. Emulsionen vom Typ Öl-in-Wasser (O/W) nach Anspruch 1, enthaltend :

   - eine Ölphase (Phase A), bestehend aus einer, gegebenenfalls mehreren, weitgehend wasserunlöslichen Komponente(n),
   - eine Wasserphase (Phase B), die gegebenenfalls weitere wasserlösliche Komponenten wie organische Verbindungen wie Alkohole, Carbonsäuren oder andere Verbindungen enthalten kann
   - an der Grenzfläche Öl-wasser angeordnete pyrogene Kieselsäure, die in der Weise teilsilyliert ist, dass der Gehalt an nicht-silylierten Oberflächensilanolgruppen an der Siliciumdioxidoberfläche sich zwischen maximal 95% und minimal 50% des Ausgangs-Siliciumdioxids bewegt, gleichbedeutend mit 1,7 bis 0,9 SiOH-Gruppen pro $nm^2$ Kieselsäureoberfläche, der Dispersions-Anteil der Oberflächenenergie gamma-s-D 30 bis 80 $mJ/m^2$ beträgt, sowie die spezifische BET-Oberfläche einen Wert von 30 bis 500 $m^2/g$ aufweist,
   - ein Elektrolytgehalt in der wäßrigen Phase, der so eingestellt ist , daß die Ionenstärke I der Lösung größer $10^{-6}$ mol/l ist, wobei die Ionenstärke definiert ist als $I = ½*\Sigma c_i*z_i^2$, wobei $c_i$ dabei die Konzentration des Ions i in der Lösung, $z_i$ die Ladung des Ions i ist,
   - die mittlere Partikelgröße der dispersen Phase, d.h. der mittlere Tropfendurchmesser, gemessen mittels Laserbeugung, 0,5 $\mu$m bis 500 $\mu$m beträgt
   - wobei die Emulsionen bei einer Leitfähigkeit der Emulsion größer oder gleich 1 $\mu S \cdot cm^{-1}$ eine Viskosität größer als 0,001 Pas aufweisen,
   - und gegebenenfalls weitere Stoffe, wie Pigmente oder Konservierungsmittel.

3. Emulsion nach Anspruch 2, **dadurch gekennzeichnet, dass** die Elektrolytkonzentration größer $10^{-6}$ mol/l ist.

4. Emulsion nach Anspruch 2 oder 3, **dadurch gekennzeichnet, dass** der Elektrolyt ein Alkali- oder Erdalkalisalz ist.

5. Emulsion nach Anspruch 4, **dadurch gekennzeichnet, dass** der Elektrolyt ein Natrium-oder Calciumsalz ist.

6. Verwendung der Emulsionen nach einem oder mehreren der Ansprüche 1 bis 5 als Beschichtungs-, Kleb- und Dichtstoffe, Emulsionen für kosmetische und pharmazeutische Anwendungen, Putz- und Reinigungsmittel oder Anwendungen zur Veränderung der Grenzflächeneigenschaften von festen und flüssigen Substraten, wie Hydrophobiermitteln, Haftvermittlern, Trennmitteln, Papierbeschichtungen oder Schaumkontrollmittel sowie zur Herstellung von W/O/W- bzw. O/W/O -Mehrfachemulsionen als Controll-Release-Systeme oder zur Segregation von inerten und reaktiven Stoffen.

7. Beschichtungs-, Kleb- und Dichtstoffe, die Emulsionen nach einem oder mehreren der Ansprüche 1 bis 5 enthalten.

8. Putz- und Reinigungsmittel, die Emulsionen nach einem oder mehreren der Ansprüche 1 bis 5 enthalten.

9. Hydrophobiermittel, Haftvermittler, Trennmittel, Papierbeschichtungen oder Schaumkontrollmittel, die Emulsionen nach einem oder mehreren der Ansprüche 1 bis 5 enthalten.

10. W/O/W- bzw. O/W/O-Mehrfachemulsionen, die Emulsionen nach einem oder mehreren der Ansprüche 1 bis 5 enthalten.

## Claims

1. Emulsions obtainable by a process in which a highly concentrated finely divided dispersion of the corresponding

silica in the liquid which forms the homogeneous phase in the emulsion is prepared in a first step, and a highly viscous preemulsion which comprises the total amount of the disperse phase and the highly concentrated finely divided dispersion of the silica, prepared in the first step, in the liquid which forms the homogeneous phase in the emulsion according to the invention is prepared in a second step, the volume of dispersion used being such that the total amount of sinter-aggregated silica required is present, and the remaining homogeneous phase is slowly metered in in a third step and an electrolyte is metered in in any one step of the steps.

**2.** Emulsions of the oil-in-water (O/W) type according to claim 1 containing:

- an oil phase (phase A), consisting of one substantially water-insoluble component or optionally a plurality of substantially water-insoluble components,
- a water phase (phase B) which may optionally contain further water-soluble components, such as organic compounds, such as alcohols, carboxylic acids or other compounds,
- pyrogenic silica which is arranged at the oil-water interface and is partly silylated in a manner such that the content of non-silylated surface silanol groups on the silica surface is from not more than 95% to not less than 50% of the starting silica, equivalent to from 1.7 to 0.9 SiOH groups per $nm^2$ of silica surface, the dispersion fraction of the surface energy gamma-s-D is from 30 to 80 $mJ/m^2$ and the specific BET surface area has a value of from 30 to 500 $m^2/g$,
- an electrolyte content in the aqueous phase, which is set such that the ionic strength I of the solution is greater than $10^{-6}$ mol/l, where the ionic strength is defined as $I = ½ \cdot \Sigma c_i \cdot z_i^2$, where $c_i$ in this case is the concentration of the ion i in the solution, $z_i$ is the charge of the ion i,
- the mean particle size of the disperse phase, i.e. the mean drop diameter, measured by means of laser diffraction, is from 0.5 $\mu m$ to 500 $\mu m$,
- the emulsions having a viscosity greater than 0.001 Pas at a conductivity of the emulsion which is greater than or equal to 1 $\mu S \cdot cm^{-1}$,
- and optionally further substances, such as pigments or preservatives.

**3.** Emulsion according to claim 2, **characterized in that** the electrolyte concentration is greater than $10^{-6}$ mol/l.

**4.** Emulsion according to claim 2 or 3, **characterized in that** the electrolyte is an alkali metal or alkaline earth metal salt.

**5.** Emulsion according to claim 4, **characterized in that** the electrolyte is a sodium or calcium salt.

**6.** Use of the emulsions according to one or more of claims 1 to 5 as coating materials, adhesives and sealants, emulsions for cosmetic and pharmaceutical applications, cleaning and cleansing agents or applications for changing the interfacial properties of solid and liquid substrates, such as water repellents, adhesion promoters, release agents, paper coatings or foam control agents and for the preparation of W/O/W or O/W/O multiple emulsions as control release systems or for the segregation of inert and reactive substances.

**7.** Coating materials, adhesives and sealants which contain emulsions according to one or more of claims 1 to 5.

**8.** Cleaning and cleansing agents which contain emulsions according to one or more of claims 1 or 5.

**9.** Water repellents, adhesion promoters, release agents, paper coatings or foam control agents which contain emulsions according to one or more of claims 1 to 5.

**10.** W/O/W or O/W/O multiple emulsions which contain emulsions according to one or more of claims 1 to 5.

## Revendications

**1.** Émulsions, pouvant être obtenues par un procédé, selon lequel lors d'une première étape, une dispersion fine hautement concentrée de la silice correspondante dans le liquide, qui forme la phase homogène dans l'émulsion est préparée, et, lors d'une deuxième étape, une pré-émulsion hautement visqueuse est préparée, qui comporte la quantité totale de la phase dispersée et la dispersion fine hautement concentrée de la silice dans le liquide fabriquée à la première étape, qui forme la phase homogène dans l'émulsion selon l'invention, le volume de dispersion utilisé étant déterminé de manière à contenir la quantité totale de silice agrégée par frittage nécessaire, et, lors d'une troisième étape, la phase homogène résiduelle est introduite lentement et, lors d'une étape quelconque parmi les

étapes, un électrolyte est introduit.

2. Émulsions de type huile dans eau (H/E) selon la revendication 1, qui contiennent :

- une phase huileuse (phase A), constituée d'un, éventuellement de plusieurs, composants sensiblement insolubles dans l'eau,
- une phase aqueuse (phase B), qui peut éventuellement contenir des composants solubles dans l'eau supplémentaires, tels que des composés organiques tels que des alcools, des acides carboxyliques ou d'autres composés,
- de la silice pyrogénée placée à l'interface huile-eau, qui est partiellement silylée de manière à ce que la teneur en groupes silanol de surface non silylés à la surface du dioxyde de silicium soit comprise entre au maximum 95 % et au minimum 50 % du dioxyde de silicium final, ce qui équivaut à 1,7 à 0,9 groupes SiOH par $nm^2$ de la surface de la silice, à ce que le taux de dispersion de l'énergie de surface gamma-s-D soit de 30 à 80 $mJ/m^2$ et à ce que la surface spécifique BET présente une valeur de 30 à 500 $m^2/g$,
- une teneur en électrolyte dans la phase aqueuse qui est ajustée de manière à ce que la force ionique I de la solution soit supérieure à $10^{-6}$ mol/l, la force ionique étant définie par $I = ½*\sum c_i*z_i^2$, $c_i$ étant la concentration de l'ion i dans la solution, $z_i$ la charge de l'ion i,
- la taille de particule moyenne de la phase dispersée, c.-à-d. le diamètre de goutte moyen, mesurée par diffraction laser, est de 0,5 $\mu$m à 500 $\mu$m,
- les émulsions présentant une viscosité supérieure à 0,001 Pas à une conductivité de l'émulsion supérieure ou égale à 1 $\mu S \cdot cm^{-1}$,
- et éventuellement des substances supplémentaires, telles que des pigments ou des conservateurs.

3. Émulsion selon la revendication 2, **caractérisée en ce que** la concentration en électrolyte est supérieure à $10^{-6}$ mol/l.

4. Émulsion selon la revendication 2 ou 3, **caractérisée en ce que** l'électrolyte est un sel alcalin ou alcalino-terreux.

5. Émulsion selon la revendication 4, **caractérisée en ce que** l'électrolyte est un sel de sodium ou de calcium.

6. Utilisation des émulsions selon une ou plusieurs des revendications 1 à 5 en tant qu'agents de revêtements, agents adhésifs et agents d'étanchéité, émulsions pour applications cosmétiques et pharmaceutiques, agents de nettoyage et de lavage ou dans des applications pour la modification des propriétés de surface de substrats solides et liquides, tels que des agents hydrophobants, des agents adhésifs, des agents démoulants, des revêtements papiers ou des agents pour le contrôle de la mousse, ainsi que pour la fabrication d'émulsions multiples E/H/E ou H/E/H, en tant que systèmes de libération contrôlée ou pour la ségrégation de substances inertes et réactives.

7. Agents de revêtements, agents adhésifs et agents d'étanchéité, qui contiennent les émulsions selon une ou plusieurs des revendications 1 à 5.

8. Agents de nettoyage et de lavage, qui contiennent les émulsions selon une ou plusieurs des revendications 1 à 5.

9. Agents hydrophobants, agents adhésifs, agents démoulants, revêtements papiers ou agents pour le contrôle de la mousse, qui contiennent les émulsions selon une ou plusieurs des revendications 1 à 5.

10. Émulsions multiples E/H/E ou H/E/H, qui contiennent les émulsions selon une ou plusieurs des revendications 1 à 5.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19842787


### In der Beschreibung aufgeführte Nicht-Patentliteratur


- **R. de Rooij ; A. A. Potanin ; D. van den Ende ; J. Mellema.** *J. Chem. Phys.,* 1993, vol. 99, 9213
- **D R Lloyd ; Th C Ward ; H P Schreiber.** Inverse Gaschromatography''-''Characterisation of Polymers and other Materials. ACS, 1989, vol. 391, 248-261
- **G.W. Sears.** *Anal. Chem,* 1950, vol. 28 (12
- **Washburn, E.W.** *Phys. Rev.,* 1921, vol. 17, 273
- **R. Lucas.** *Kolloid Z.,* 1918, vol. 23, 15
- **Schoelkopf et al.** *J. Colloid. Interf. Sci.,* 2000, vol. 227, 119-131
- **D R Lloyd ; Th C Ward ; H P Schreiber.** Characterisation of Polymers and other Materials. ACS Symposium Series, 1989, vol. 391, 248-261